(19) Europäisches Patentamt / European Patent Office / Office européen des brevets

(11) **EP 4 146 350 B1**

(12) **EUROPEAN PATENT SPECIFICATION**

(45) Date of publication and mention
of the grant of the patent:
**08.11.2023 Bulletin 2023/45**

(21) Application number: **21721936.9**

(22) Date of filing: **29.04.2021**

(51) International Patent Classification (IPC):
**A61Q 17/00** (2006.01)  **A61Q 19/10** (2006.01)
**A61K 8/19** (2006.01)  **A61K 8/23** (2006.01)
**A61K 8/36** (2006.01)  **A61K 8/365** (2006.01)
**A61K 8/368** (2006.01)  **A61K 8/46** (2006.01)
**A61K 8/81** (2006.01)

(52) Cooperative Patent Classification (CPC):
**A61Q 17/005; A61K 8/19; A61K 8/23; A61K 8/36;**
**A61K 8/361; A61K 8/365; A61K 8/368;**
**A61K 8/463; A61K 8/8152; A61Q 19/10;**
**Y02A 50/30**

(86) International application number:
**PCT/EP2021/061323**

(87) International publication number:
**WO 2021/224114 (11.11.2021 Gazette 2021/45)**

(54) **ANTIBACTERIAL COMPOSITION**

ANTIBAKTERIELLE ZUSAMMENSETZUNG

COMPOSITION ANTIBACTÉRIENNE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.05.2020 EP 20172648**

(43) Date of publication of application:
**15.03.2023 Bulletin 2023/11**

(73) Proprietors:
• **Unilever IP Holdings B.V.**
**3013 AL Rotterdam (NL)**
Designated Contracting States:
**AL AT BE BG CH CZ DK EE ES FI FR GR HR HU
IS LI LT LU LV MC MK NL NO PL PT RO SE SI SK
SM**
• **Unilever Global IP Limited**
**Wirral, Merseyside CH62 4ZD (GB)**
Designated Contracting States:
**CY DE GB IE IT MT RS TR**

(72) Inventors:
• **CARNALI, Joseph, Oreste**
**Trumbull, Connecticut 06611 (US)**
• **CHANDAR, Prem**
**Trumbull, Connecticut 06611 (US)**
• **WALSH, Connor, Patrick**
**Trumbull, Connecticut 06611 (US)**

(74) Representative: **Fijnvandraat, Arnoldus**
**Unilever Patent Group**
**Bronland 14**
**6708 WH Wageningen (NL)**

(56) References cited:
WO-A1-2014/170187   WO-A1-2017/067852
WO-A1-2017/084855   WO-A1-2017/084867
WO-A2-2004/028461   US-A1- 2011 224 120
US-A1- 2012 034 314   US-A1- 2016 053 206
US-A1- 2016 362 646   US-B1- 6 294 186

EP 4 146 350 B1

## Description

### Field of the invention

Disclosed herein are antimicrobial cleansing compositions. The antimicrobial cleansing compositions can include 5 to 25% by weight of a sulfate surfactant; 0 to 5% by weight of a fatty acid soap; 0.03 to 3 parts per million of a silver compound; 0.05 to 1% by weight of a thickening agent; and 1 to 10% by weight of a nitrate. A pH of the cleansing composition is 5 to 10. The antimicrobial cleansing compositions can also include 5 to 25% by weight of a sulfate surfactant 0.03 to 3 parts per million of a silver compound; 0.05 to 1% by weight of a thickening agent; 1 to 10% by weight of a nitrate; and 0.05 to 1% by weight of a preservative. The pH of the cleansing composition is 4 to 5.5.

### Background of the invention

As the largest organ of the human body, the skin performs several major functions. It serves as a boundary layer, helping to maintain body temperature and retain essential fluids. It also protects against toxic agents and harmful solar radiation. Human skin is exposed daily to a range of microbial contaminants via exposure to bodily fluids and various environmental factors. These contaminants include Gram-negative and Gram-positive bacteria, yeast, fungi, mold, protozoan, and viruses. Once established on skin, these organisms can cause inflammation, irritation, and/or infection. Irritation can initiate an elaborate cascade of immunological events. Thus, proper skin maintenance is essential for good health and begins, for many people, with daily cleansing. Regular skin cleansing can reduce the levels of microbial contaminants on the skin and so can also prevent or minimize skin irritation and inflammation (D.W. Koenig et al., "Skin cleansing composition incorporation anionic particles", US 6,764,988).

Traditionally, skin cleansing includes any activity that kills, binds, or removes contaminants on or from the skin's surface and is often accomplished using a solution-based "liquor" generated from a liquid cleansing product or a cleansing solid such as a soap bar. While removal of microbial contaminants does occur as a result of these activities, the level of contamination is typically only reduced by a factor of 10 to 100 (one to two $\log_{10}$ reduction). The residual level of microbes is then often sufficient to quickly re-establish the microbial contamination. For this reason, it is often more attractive to employ a biocidal active ingredient in the cleansing product to achieve a more profound reduction in the microbial population.

The mechanism of action of antimicrobial agents is relevant to the danger of an organism developing a resistance to the agent. Agents which work on a single, relatively obscure, critical site on/in the organism are more easily circumvented, requiring only a single mutation to develop resistance. On the other hand, agents which target a number of sites, interfering with basic and indispensable cellular processes, are less likely to be defeated by a few random mutations (D.G. Romero-Urbina et al., "Ultrastructural change in methicillin-resistant Staphylococcus aureus induced by positively charged silver nanoparticles", Beilstein Journal of Nanotechnology 6, 2396-2405 (2015).

The use of silver as a biocidal agent has been previously discussed (R.E. Burrell, "A scientific perspective on the use of topical silver preparations", Ostomy/Wound Management 49, 19-24 (2003); A.B.G. Lansdown and A. Williams, "How safe is silver in wound care", J. Wound Care 13, 131-136 (2004)) and is believed to involve multiple lines of attack, including damaging the bacterial membrane and cell wall, coagulation of the cellular components, binding and condensation of DNA, and inactivation of critical membrane-bound proteins and enzymes via binding with thiol groups (Q.L. Feng et al., "A mechanistic study of the antibacterial effect of silver ions on Escherichia coli and Staphylococcus aureus", J. Biomedical Materials Research 52, 662-668 (2000)). Thus, silver ion is less likely to be thwarted by a random mutation in an organism. However, the time scale for antimicrobial action by silver ion alone is not really suitable for a handwash application. The conditions relevant to hand-washing place restrictions on both the concentration of active ingredients and the contact time with contaminated surfaces. Personal experience will suggest that about 30 to about 60 seconds are upper limits to the time typically taken for hand-washing (P. Chandar et al., "Liquid soap having enhanced antibacterial activity", US 2016/0053206 A1). However, most studies of the biocidal action of silver ion are conducted on a time scale of many minutes to hours (C.P. Randall et al., "The silver cation (Ag+): anti-staphylococcal activity, mode of action and resistance studies", J. Antimicrobial Chemotherapy 68, 131-138 (2013)). This relatively measured pace is especially true for Gram-positive bacteria, such as *Staphylococcus aureus,* where the thick peptidoglycan layer containing negatively charged binding sites is thought to slow the action of silver ions through the bacterial cell wall. It is advantageous to somehow accelerate the biocidal action of silver, so that it could be useful in a cleansing product. Problems with discoloration due to formation of silver oxides would be minimized if the silver levels employed could also be reduced (Agarkhed et al., "Cleansing composition containing oligodynamic metal and efficacy enhancing agent", US 2016/0362646 A1).

A solution meeting the challenges of rapid biocidal action with minimal silver ion levels has recently been disclosed, consisting of combining fatty acid soaps with silver ion. The resulting mixture shows a considerable enhancement in biocidal action over that of the two ingredients taken separately (Chandar et al., US 2016/0053206 A1). However, this synergy has previously been thought to be limited to combinations of fatty soaps with silver (at soap-like pH's in the range of 10-11) and to not extend to non-soap surfactants and to lower pH ranges.

It is continually desired to provide a cleansing composition that provides the desired biocidal action at lower pHs with the possibility of using non-soap surfactants.

## Summary of the invention

Disclosed, in various aspects, are antimicrobial cleansing compositions. An antimicrobial cleansing composition comprises: 5 to 25% by weight of a sulfate surfactant; 0 to 5% by weight of a fatty acid soap; 0.03 to 3 parts per million of a silver compound; 0.05 to 1 % by weight of a thickening agent; and 1 to 10% by weight of a nitrate. A pH of the cleansing composition is 5 to 10.

An antimicrobial cleansing composition comprises: 5 to 25% by weight of a sulfate surfactant; 0.03 to 3 parts per million of a silver compound; 0.05 to 1% by weight of a thickening agent; 1 to 10% by weight of a nitrate; and 0.05 to 1% by weight of a preservative. A pH of the cleansing composition is 4 to 5.5.

The above described and other features and characteristics are exemplified by the following detailed description.

## Detailed description of the invention

Disclosed herein are various antimicrobial cleansing compositions. The presently disclosed antimicrobial cleansing compositions disclose that the class of sulfate surfactants also show enhanced biocidal action with silver and does so over a much wider pH range, from pH 4 to pH 10, for example, 5 to 10, for example 4 to 5.5, for example, 4.5 to 5.5. At the lower end of this pH range, the corresponding skin cleansing formulations are compatible with various preservatives, such as organic acids, including, but not limited to, benzoic acid, sorbic acid, or a combination thereof. Without wishing to be limited by theory, it is likely that the effectiveness of this enhanced biocidal action has not been previously recognized because the high levels of chloride ion usually found in such skin cleansing formulations tend to precipitate silver ion as silver chloride. Such high levels can be present in formulations as byproducts of surfactant synthesis or purposefully added to adjust formulation rheology. It was unexpectedly discovered with the compositions disclosed herein that the replacement of chloride salts by nitrate salts will maintain the solubility and efficacy of silver ion while still allowing for rheology manipulation. Nitrate salts will also provide the synergy with silver with a degree of protection against chloride ion and against polycarboxylates.

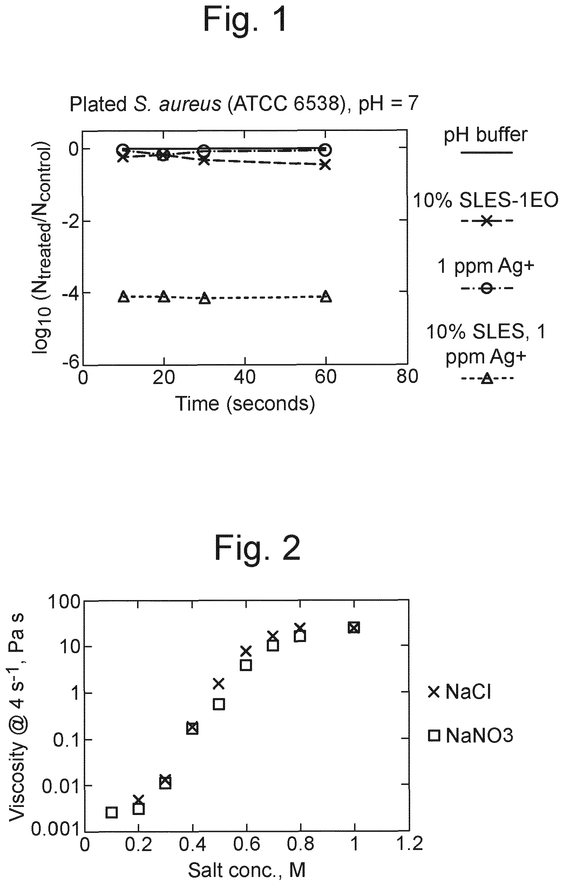

The synergy suggested above is best described by Figure 1, which shows the results of a Time-Kill experiment in which an inoculum of *S. aureus* is exposed to a series of agents for up to 60 seconds (s) and the surviving organisms isolated and enumerated. Figure 1 shows the enhanced biocidal action when the surfactant SLES-1 EO is combined with silver ion. Inoculums of *S. aureus* at about $10^{7.5}$ colony forming units per milliliter (CFU/mL) were prepared from 24-hour cultures on agar plates. Conditions of the test were pH 7 and 25 °C.

The $\log_{10}$ of (number of surviving cells (CFU/mL)/initial number of cells) is plotted versus the exposure time. The symbol size matches or exceeds the magnitude of the error bar and the surviving number of cells in the case of SLES plus silver ion is at the detection limit of 2000 CFU/mL. The exposure time is limited to only 60 s, as this time frame is a realistic estimate of the amount of time devoted to hand washing. It is observed that a control wash with water has no biocidal effect under these conditions, and neither does a typical level of anionic surfactant (a model 10% SLES-1 EO-based formulation diluted 1:1 with water). Also shown is a wash with 1 ppm silver ion delivered from a silver nitrate solution, the pH of this and the preceding solutions being adjusted to 7 using diluted NaOH and $HNO_3$ solutions. Again, the silver solution shows no biocidal effect on this short time-scale exposure. Lastly, the combination of 10% SLES and 1 ppm silver ion diluted 1:1 with water shows a 4 $\log_{10}$ reduction in surviving organism after an exposure time of as little as 10 s. Note that this level of reduction represents the limit of detection under these experimental conditions.

While anionic surfactants can have biocidal action against certain microbes and silver ion is well recognized as having broad spectrum, multi-dimensional biocidal action, their individual efficacies at the levels and time scales tested in Figure 1 are not remarkable. However, their combination is seen to result in a truly remarkable, synergistic biocidal effect which acts on a time scale useful for body cleansing.

A cleansing composition as disclosed herein can include a sulfate surfactant, a fatty acid soap, a silver compound, a thickening agent and a nitrate. A pH of this cleansing composition is 5 to 10, for example, 6 to 9. A cleansing composition can include a sulfate surfactant, a silver compound, a thickening agent, a nitrate, and a preservative. A pH of this cleansing composition is 4 to 5.5, for example, 4.5 to 5.5.

The components for the antimicrobial cleansing composition can be present in various amounts. For example, the sulfate surfactant can be present in an amount of 5 to 25% by weight, for example, 10 to 20% by weight. The silver compound can be present in an amount of 0.03 to 3 parts per million.

The nitrate can be present in an amount of 1 to 10% by weight, for example, 2.5 to 7.5% by weight. The thickening agent can be present in an amount of 0.05 to 1% by weight. The fatty acid can be present in an amount of 0 to 5% by weight, for example, 0.01 to 4% by weight, for example, 0.05 to 3% by weight, for example, 0.1 to 2.5% by weight. The preservative can be present in an amount of 0.05 to 1% by weight, for example, 0.07 to 0.9% by weight.

The sulfate surfactant can be an anionic surfactant. The sulfate surfactant can contain $C_8$-$C_{18}$ alkyl groups, for example, $C_{12}$-$C_{16}$ alkyl groups, for example, $C_{10}$-$C_{14}$ alkyl groups, or mixtures thereof. For example, the sulfate surfactant can contain $C_{10}$ alkyl groups, $C_{12}$ alkyl groups, $C_{14}$ alkyl groups, or any combination thereof. The sulfate surfactant can contain partially unsaturated alkyl groups. For example, the sulfate surfactant can contain $C_{18:1}$ or $C_{18:2}$ alkenyl groups. For example, the surfactant can be selected from alkyl sulfate, alkyl ether sulfate, or a combination thereof. The surfactant can preferably be sodium lauryl ether sulfate, or more preferably, the surfactant can be sodium lauryl ether sulfate with 0, 1, 2, or 3 ethoxy groups. SLES-1EO generally refers to sodium lauryl ether sulfate with (on average) 1 ethoxy group, SLES-2EO generally refers to sodium lauryl ether sulfate with (on average) 2 ethoxy groups, and SLES-3EO generally refers to sodium lauryl ether sulfate with (on average) 3 ethoxy groups.

As to the anionic surfactant present in the cleansing composition disclosed herein, the anionic surfactant used can include aliphatic sulfonates, such as a primary alkane (e.g., $C_8$-$C_{22}$) sulfonate, primary alkane (e.g., $C_8$-$C_{22}$) disulfonate, $C_8$-$C_{22}$ alkene sulfonate, Cs-$C_{22}$ hydroxyalkane sulfonate or alkyl glyceryl ether sulfonate (AGS); or aromatic sulfonates such as alkyl benzene sulfonate. The anionic may also be an alkyl sulfate (e.g., $C_{12}$-$C_{18}$ alkyl sulfate) or alkyl ether sulfate (including alkyl glyceryl ether sulfates). Among the alkyl ether sulfates are those having the formula:

$$RO(CH_2CH_2O)nSO_3M$$

wherein R is an alkyl or alkenyl having 8 to 18 carbons, preferably 12 to 18 carbons, n has an average value of at least 1.0, preferably less than 5, and most preferably 1 to 4, and M is a solubilizing cation such as sodium, potassium, ammonium, or substituted ammonium.

The anionic surfactant may also be alkyl sulfosuccinates (including mono- and dialkyl, e.g., $C_6$-$C_{22}$ sulfosuccinates); alkyl and acyl taurates (often methyl taurates), alkyl and acyl sarcosinates, sulfoacetates, $C_8$-$C_{22}$ alkyl phosphates and phosphonates, alkyl phosphate esters and alkoxyl alkyl phosphate esters, acyl lactates, $C_8$-$C_{22}$ monoalkyl succinates and maleates, sulphoacetates, alkyl glucosides and acyl isethionates.

Sulfosuccinates may be monoalkyl sulfosuccinates having the formula:

$$R^1OC(O)CH_2CH(SO_3M)CO_2M;$$

and amide-MEA sulfosuccinates of the formula:

$$R^1CONHCH_2CH_2OC(O)CH_2CH(SO_3M)CO_2M$$

wherein $R^1$ ranges from $C_8$-$C_{22}$ alkyl.

Sarcosinates are generally indicated by the formula:
$R^2CON(CH_3)CH_2CO_2M$, wherein $R^2$ ranges from $C_8$-$C_{20}$ alkyl.

Taurates are generally identified by formula:

$$R^3CONR^4CH_2CH_2SO_3M$$

wherein $R^3$ is a $C_8$-$C_{20}$ alkyl, $R^4$ is a $C_1$-$C_4$ alkyl.

M is a solubilizing cation as previously described.

The cleansing composition disclosed herein may contain $C_8$-$C_{18}$ acyl isethionates. These esters are prepared by a reaction between alkali metal isethionate with mixed aliphatic fatty acids having from 6 to 18 carbon atoms and an iodine value of less than 20. At least 75% of the mixed fatty acids have from 12 to 18 carbon atoms and up to 25% have from 6 to 10 carbon atoms.

The acyl isethionate may be an alkoxylated isethionate such as is described in Ilardi et al., U.S. Pat. No. 5,393,466, entitled "Fatty Acid Esters of Polyalkoxylated isethonic acid; issued Feb. 28, 1995. This compound has the general formula:

$$R^5C-(O)O-C(X)H-C(Y)H-(OCH_2-CH_2)_m-SO_3M$$

wherein $R^5$ is an alkyl group having 8 to 18 carbons, m is an integer from 1 to 4, X and Y are each independently hydrogen or an alkyl group having 1 to 4 carbons and M is a solubilizing cation as previously described.

In an aspect of the cleansing composition, the anionic surfactant used is 2-acrylamido-2-methylpropane sulfonic acid, ammonium lauryl sulfate, ammonium perfluorononanoate, potassium lauryl sulfate, sodium alkyl sulfate, sodium dodecyl sulfate, sodium laurate, sodium laureth sulfate, sodium lauroyl sarcosinate, sodium stearate, sodium sulfosuccinate esters, or a combination thereof. Such anionic surfactants are commercially available from suppliers like Galaxy Surfactants, Clariant, Sino Lion, Stepan Company, and Innospec.

Optionally, amphoteric surfactants can be included in the cleansing compositions disclosed herein. Amphoteric surfactants (which depending on pH can be zwitterionic) include sodium acyl amphoacetates, sodium acyl amphopropionates, disodium acyl amphodiacetates and disodium acyl amphodipropionates where the acyl (i.e., alkanoyl group) can comprise a $C_7$-$C_{18}$ alkyl portion. Illustrative examples of amphoteric surfactants include sodium lauroamphoacetate, sodium cocoamphoacetate, sodium lauroamphoacetate, or a combination thereof.

As to the zwitterionic surfactants employed in the present cleansing composition, such surfactants include at least one acid group. Such an acid group may be a carboxylic or a sulphonic acid group. They often include quaternary nitrogen, and therefore, can be quaternary amino acids. They should generally include an alkyl or alkenyl group of 7 to 18 carbon atoms and generally comply with an overall structural formula:

$$R^6\text{-}[\text{-}C(O)\text{-}NH(CH_2)_q\text{-}]_r\text{-}N^+(R^7)(R^8)\text{-}A\text{-}B$$

where $R^6$ is alkyl or alkenyl of 7 to 18 carbon atoms; $R^7$ and $R^8$ are each independently alkyl, hydroxyalkyl or carboxyalkyl of 1 to 3 carbon atoms; q is 2 to 4; r is 0 to 1; A is alkylene of 1 to 3 carbon atoms optionally substituted with hydroxyl, and B is $\text{-}CO_2\text{-}$ or $\text{-}SO_3\text{-}$.

Desirable zwitterionic surfactants for use in the cleansing composition disclosed herein and within the above general formula include simple betaines of formula:

$$R^6\text{-}N^+(R^7)(R^8)\text{-}CH_2CO_2^-$$

and amido betaines of formula:

$$R^6\text{-}CONH(CH_2)_t\text{-}N^+(R^7)(R^8)\text{-}CH_2CO_2^-$$

where t is 2 or 3.

In both formulae $R^6$, $R^7$ and $R^8$ are as defined previously. $R^6$ may, in particular, be a mixture of $C_{12}$ and $C_{14}$ alkyl groups derived from coconut oil so that at least half, preferably at least three quarters of the groups $R^6$ have 10 to 14 carbon atoms. $R^7$ and $R^8$ are preferably methyl.

A further possibility is that the zwitterionic surfactant is a sulphobetaine of formula:

$$R^6\text{-}N^+(R^7)(R^8)\text{-}(CH_2)_3SO_3^-$$

or

$$R^6\text{-}CONH(CH_2)_u\text{-}N^+(R^7)(R^8)\text{-}(CH_2)_3SO_3^-$$

where u is 2 or 3, or variants of these in which $\text{-}(CH_2)_3SO_3^-$ is replaced by $\text{-}CH_2C(OH)(H)CH_2SO_3^-$.

In these formulae, $R^6$, $R^7$ and $R^8$ are as previously defined.

Illustrative examples of the zwitterionic surfactants desirable for use include betaines such as lauryl betaine, betaine citrate, cocodimethyl carboxymethyl betaine, cocoamidopropyl betaine, coco alkyldimethyl betaine, and laurylamidopropyl betaine.

An additional zwitterionic surfactant suitable for use includes cocoamidopropyl sultaine, for example, cocamidopropyl hydroxysultaine. Preferred zwitterionic surfactants include lauryl betaine, betaine citrate, sodium hydroxymethylglycinate, (carboxymethyl) dimethyl-3-[(1-oxododecyl) amino] propylammonium hydroxide, coco alkyldimethyl betaine, (carboxymethyl) dimethyloleylammonium hydroxide, cocoamidopropyl betaine, (carboxymethyl) dimethyloleylammonium hydroxide, cocoamidopropyl betaine, (carboxylatomethyl) dimethyl(octadecyl)ammonium, cocamidopropyl hydroxysultaine, or a combination thereof. Such surfactants are made commercially available from suppliers like Stepan Company, Solvay, Evonik and it is within the scope of the cleansing compositions disclosed herein to employ mixtures of the aforementioned surfactants.

Nonionic surfactants may optionally be used in the cleansing composition. When used, nonionic surfactants are typically used at levels as low as 0.5, 1, 1.5 or 2% by weight and at levels as high as 6, 8, 10 or 12% by weight. The nonionics which may be used include in particular the reaction products of compounds having a hydrophobic group and a reactive hydrogen atom, for example aliphatic alcohols, acids, amides or alkylphenols with alkylene oxides, especially ethylene oxide either alone or with propylene oxide. Specific nonionic surfactant compounds are alkyl ($C_6$-$C_{22}$) phenols, ethylene oxide condensates, the condensation products of aliphatic ($C_8$-$C_{18}$) primary or secondary linear or branched alcohols with ethylene oxide, and products made by condensation of ethylene oxide with the reaction products of propylene oxide and ethylenediamine. Other nonionic surfactants include long chain tertiary amine oxides, long chain tertiary phosphine oxides, dialkyl sulphoxides.

In an aspect, nonionic surfactants can include fatty acid/alcohol ethoxylates having the following structures a) $HOCH_2(CH_2)_s(CH_2CH_2O)_c$ H or b) $HOOC(CH_2)_v(CH_2CH_2O)_d$ H; where s and v are each independently an integer up to 18; and c and d are each independently an integer from 1 or greater. In an aspect, s and v can be each independently 6 to 18; and c and d can be each independently 1 to 30. Other options for nonionic surfactants include those having the formula $HOOC(CH_2)_i$-CH=CH-$(CH_2)_k(CH_2CH_2O)_z$ H, where i, k are each independently 5 to 15; and z is 5 to 50. In another aspect, i and k are each independently 6 to 12; and z is 15 to 35.

The nonionic surfactant may also include a sugar amide, such as a polysaccharide amide. Specifically, the surfactant may be one of the lactobionamides described in U.S. Pat. No. 5,389,279 to Au et al., entitled "Compositions Comprising Nonionic Glycolipid Surfactants issued Feb. 14, 1995; or it may be one of the sugar amides described in U.S. Pat. No. 5,009,814 to Kelkenberg, titled "Use of N-Poly Hydroxyalkyl Fatty Acid Amides as Thickening Agents for Liquid Aqueous Surfactant Systems" issued Apr. 23, 1991.

Illustrative examples of nonionic surfactants that can optionally be used in the cleansing compositions disclosed herein include, but are not limited to, polyglycoside, cetyl alcohol, decyl glucoside, lauryl glucoside, octaethylene glycol monododecyl ether, n-octyl beta-d-thioglucopyranoside, octyl glucoside, oleyl alcohol, polysorbate, sorbitan, stearyl alcohol, or a combination thereof.

In an aspect, cationic surfactants may optionally be used in the cleansing composition of the present application.

One class of cationic surfactants includes heterocyclic ammonium salts such as cetyl or stearyl pyridinium chloride, alkyl amidoethyl pyrrylinodium methyl sulfate, and lapyrium chloride.

Tetra alkyl ammonium salts are another useful class of cationic surfactants for use. Examples include cetyl or stearyl trimethyl ammonium chloride or bromide; hydrogenated palm or tallow trimethylammonium halides; behenyl trimethyl ammonium halides or methyl sulfates; decyl isononyl dimethyl ammonium halides; ditallow (or distearyl) dimethyl ammonium halides, and behenyl dimethyl ammonium chloride.

Still other types of cationic surfactants that may be used are the various ethoxylated quaternary amines and ester quats. Examples include PEG-5 stearyl ammonium lactate (e.g., Genamin KSL manufactured by Clariant), PEG-2 coco ammonium chloride, PEG-15 hydrogenated tallow ammonium chloride, PEG 15 stearyl ammonium chloride, dipalmitoyl ethyl methyl ammonium chloride, dipalmitoyl hydroxyethyl methyl sulfate, and stearyl amidopropyl dimethylamine lactate.

Still other useful cationic surfactants include quaternized hydrolysates of silk, wheat, and keratin proteins, and it is within the scope of the cleansing composition to use mixtures of the aforementioned cationic surfactants.

If used, cationic surfactants will make up no more than 1.0% by weight of the cleansing composition. When present, cationic surfactants typically make up from 0.01 to 0.7%, and more typically, from 0.1 to 0.5% by weight of the cleansing composition, including all ranges subsumed therein.

The silver compound can comprise a silver ion, for example, the silver ion can be selected from silver nitrate, silver acetate, silver oxide, silver sulfate, or a combination thereof. Preferably, the silver compound is silver nitrate.

Stated more specifically, the silver compounds employed in the subject formulations and compositions are one or more water-soluble silver (I) compounds having a silver ion solubility of at least $1.0\times10^{-4}$ mol/L (in water at 25 °C). Silver ion solubility, as referred to herein, is a value derived from a solubility product (Ksp) in water at 25 °C, a well-known parameter that is reported in numerous sources. More particularly, silver ion solubility [Ag+], a value given in mol/L may be calculated using the formula:

$$[Ag+]=(Ksp\cdot x)^{(1/(x+1))},$$

wherein Ksp is the solubility product of the compound of interest in water at 25 °C, and x represents the number of moles of silver ion per mole of compound. It has been found that silver (I) compounds having a silver ion solubility of at least $1\times10^{-4}$ mol/L are desirable for use herein. Silver ion solubility values for a variety of silver compounds are given in Table 1.

Table 1: Silver Ion Solubility

| Silver Compound | X | Ksp (mol/L in water at 25°C) | Silver Ion Solubility [Ag+] (mol/L in water at 25°C) |
|---|---|---|---|
| | | | |
| Silver nitrate | 1 | 51.6 | 7.2 |
| Silver acetate | 1 | $2.0 \times 10^{-3}$ | $4.5 \times 10^{-2}$ |
| Silver sulfate | 2 | $1.4 \times 10^{-5}$ | $3.0 \times 10^{-2}$ |
| Silver benzoate | 1 | $2.5 \times 10^{-5}$ | $5.0 \times 10^{-3}$ |

(continued)

| Silver Compound | X | Ksp (mol/L in water at 25°C) | Silver Ion Solubility [Ag+] (mol/L in water at 25°C) |
|---|---|---|---|
| Silver salicylate | 1 | $1.5 \times 10^{-5}$ | $3.9 \times 10^{-3}$ |
| Silver carbonate | 2 | $8.5 \times 10^{-12}$ | $2.6 \times 10^{-4}$ |
| Silver citrate | 3 | $2.5 \times 10^{-16}$ | $1.7 \times 10^{-4}$ |
| Silver oxide | 1 | $2.1 \times 10^{-8}$ | $1.4 \times 10^{-4}$ |
| Silver phosphate | 3 | $8.9 \times 10^{-17}$ | $1.3 \times 10^{-4}$ |
| Silver chloride | 1 | $1.8 \times 10^{-10}$ | $1.3 \times 10^{-5}$ |
| Silver bromide | 1 | $5.3 \times 10^{-13}$ | $7.3 \times 10^{-7}$ |
| Silver iodide | 1 | $8.3 \times 10^{-17}$ | $9.1 \times 10^{-9}$ |
| Silver sulfide | 2 | $8.0 \times 10^{-51}$ | $2.5 \times 10^{-17}$ |

Among the silver compounds desirable for use herein are silver oxide, silver nitrate, silver acetate, silver sulfate, silver benzoate, silver salicylate, silver carbonate, silver citrate and silver phosphate, with silver nitrate, silver acetate, silver oxide, silver sulfate, or a combination thereof of particular interest. In at least one preferred embodiment the silver compound comprises silver nitrate.

In at least one embodiment the silver compound is not silver dihydrogen citrate. In another embodiment the silver compound is not a salt of alginic acid or substantially depolymerized alginic acid. In one preferred embodiment the silver compound is not in the form of nano particles, attached to nano particles or part of intercalated silicates such as, for example, bentonite.

In one or more embodiments, the ratio by weight of silver compound to surfactant in the aqueous soap compositions employed in the subject methods is from 1:5000 to 1:6,500,000, preferably from 1:10,000 to 1:2,500,000, more preferably from 1:12,000 to 1 :2,000,000.

The cleansing composition disclosed herein can further include a thickening agent. Particularly preferred thickening agents include an anionic polymer-based thickener, preferably, for example, a polyacrylate based polymer or copolymer, or a combination thereof. Polysaccharides can be used. Examples include fibers, starches, natural/synthetic gums and cellulosics. Representative of the starches are chemically modified starches such as sodium hydroxypropyl starch phosphate and aluminum starch octenylsuccinate. Tapioca starch is often preferred, as is maltodextrin. Suitable gums include xanthan, sclerotium, pectin, karaya, arabic, agar, guar (including Acacia senegal guar), carrageenan, alginate and combinations thereof. Suitable cellulosics include hydroxypropyl cellulose, hydroxypropyl methylcellulose, ethylcellulose, sodium carboxy methylcellulose (cellulose gum/carboxymethyl cellulose) and cellulose (e.g. cellulose microfibrils, cellulose nanocrystals or microcrystalline cellulose).

Sources of cellulose microfibrils include secondary cell wall materials (e.g. wood pulp, cotton), bacterial cellulose, and primary cell wall materials. Preferably the source of primary cell wall material is selected from parenchymal tissue from fruits, roots, bulbs, tubers, seeds, leaves and combination thereof; more preferably is selected from citrus fruit, tomato fruit, peach fruit, pumpkin fruit, kiwi fruit, apple fruit, mango fruit, sugar beet, beet root, turnip, parsnip, maize, oat, wheat, peas and combinations thereof; and even more preferably is selected from citrus fruit, tomato fruit and combinations thereof. A most preferred source of primary cell wall material is parenchymal tissue from citrus fruit. Citrus fibers, such as those made available by Herbacel® as AQ Plus can also be used as source for cellulose microfibrils. The cellulose sources can be surface modified by any of the known methods including those described in Colloidal Polymer Science, Kalia et al., "Nanofibrillated cellulose: surface modification and potential applications" (2014), Vol 292, Pages 5-31.

As mentioned, synthetic polymers are effective thickening agents. This category includes crosslinked polyacrylates such as the Carbomers, polyacrylamides such as Sepigel® 305 and taurate copolymers such as Simulgel® EG and Aristoflex® AVC, the copolymers being identified by respective INCI nomenclature as sodium acrylate/sodium acryloyldimethyl taurate and acryloyl dimethyltaurate/vinyl pyrrolidone copolymer. Another preferred synthetic polymer suitable for thickening is an acrylate-based polymer made commercially available by Seppic and sold under the name Simulgel INS100. Calcium carbonate, fumed silica, and magnesium-aluminum-silicate may also be used.

Sodium hydroxypropyl starch phosphate, aluminum starch octenylsuccinate, tapioca starch, maltodextrin, xanthan gum, agar gum, guar gum, carrageenan gum, alginate gum, hydroxypropyl cellulose, hydroxypropyl methylcellulose, ethylcellulose, sodium carboxy methylcellulose, cellulose, polyethylene glycol (e.g., polyethylene glycol diester stearic acid), or a combination thereof are further examples of thickening agents for use in the present cleansing compositions.

Such thickening agents are commercially available from the Dow Chemical Company or the Hallstar Company.

The nitrate can comprise sodium nitrate, potassium nitrate, or a combination thereof, preferably, the nitrate comprises potassium nitrate.

The term "fatty acid soap" or, more simply, "soap" is used here in its popular sense, i.e., salts of aliphatic alkane- or alkene monocarboxylic fatty acids preferably having 6 to 22 carbon atoms, and preferably 8 to 18 carbon atoms.

The fatty acid soap comprises 0 to 5% by weight of the subject cleansing formulations. The referenced fatty acid levels are for formulations in the form typically provided to consumers, without taking into account dilution in use. In use, formulations containing fatty acid soap at higher levels within such range are typically diluted with water such that the diluted composition that is applied to the skin contains 5% or less, more particularly from 0.2 to 5% or less of fatty acid soap.

Typical of the soap salts are alkali metal or alkanol ammonium salts of such fatty acids, although other metal salts thereof, e.g., magnesium salts, may also be employed. Sodium, potassium, magnesium, mono-, di- and tri-ethanol ammonium salts of such acids are among the desirable soaps for use herein. Sodium soaps are most preferred in the cleansing compositions disclosed herein, particularly preferred is a sodium soap derived from sodium dodecanoate, sodium laurate, sodium oleate, or a combination thereof

As noted previously, the fatty acids from which the soap salts are derived may contain unsaturation. The level of unsaturation should be in accordance with commercially acceptable standards. Excessive unsaturation is normally avoided to minimize color and odor issues. Commonly, not more than to 40% by weight of the fatty acids from which the soap salts are formed are unsaturated. In one or more embodiments, from 10 to 40% by weight, more particularly from 20 to 40% by weight of the fatty acids from which the soap salts are formed are unsaturated.

Preservatives can desirably be incorporated into the cleansing composition to protect against the growth of potentially harmful microorganisms. Preservatives are antimicrobial ingredients added to maintain the microbiological safety of products. They act to inhibit the growth of microbes and so reduce the level of microbial contamination. As personal wash formulations contain biodegradable ingredients, they can become unpleasant and unsafe if microbial breakdowns is not controlled. Microbial growth is water dependent, so preservatives must partition to some extent into the aqueous phase of a formulation. Commonly used preservatives can be categorized into the following five classes:

1) Parabens such as Methyl-, Propyl-, and Butylparaben and Germaben II are derived from para-hydroxy benzoic acid. These materials are economical and effective against fungals and some Gram negative bacteria but need a second ingredient to control Gram positives. They also tend to partition more towards the oil phase in emulsion-containing formulations. They are widely employed at levels of 0.01-0.3% by weight and are generally considered safe - though there have been concerns over possible estrogenic activity and links to cancer.

2) Formaldehyde Releasers such as Germall Plus, DMDM Hydantoin, and Imadozilidinyl or Diazolidinyl Urea. This class of materials is effective against bacteria but offers only weak antifungal activity. They are used at levels of 0.1-0.5% by weight in the pH range 3-8. The low levels of free formaldehyde released ensure microbial inhibition, but cause concerns as potential carcinogens.

3) Isothiazolinones such as methylcholoroisothiazolinone (MCI), methylisothiazolinone (MI), and Kathon. Isothiazolinones offer broad spectrum effectiveness over a broad pH range, but they may cause skin irritation for some consumers. This class of materials is employed at low levels, on the order of 10's of ppms.

4) Phenoxyethanol, marketed as Optiphen or Optiphen Plus and Neolone PH 100. Phenoxyethanol is often considered as a milder alternative to parabens or formaldehyde-donors but has a narrow spectrum of applicability to Gram negative bacteria. It is generally combined with caprylyl glycol, sorbic acid/potassium sorbate, or EDTA to create broad spectrum efficacy. It is applicable over a wide range of pH, with a typical usage level of 1% or less. However, there are some concerns over possible carcinogenic activity.

5) Organic Acids such as Benzoic Acid/Sodium Benzoate, Sorbic Acid/Potassium Sorbate, Salicylic Acid/Sodium Salicylate, and Levulinic or Anisic Acids. The use of these acids is confined to aqueous applications in the pH range 2-6. They typically are used at higher levels than some of the above alternatives and have somewhat weaker efficiency against bacteria (which can be augmented by combination with diazolidinyl urea), though they are very good against fungi. This class of preservatives are generally considered as natural.

Preservatives for use in the cleansing compositions disclosed herein preferably include organic-acid based preservatives, preferably benzoic acid, salicylic acid, levulinic acid, ansic acid, or a combination thereof. Traditional preservatives for use include hydantoin derivatives and propionate salts.

Other preservatives for use are iodopropynyl butyl carbamate, phenoxyethanol, 1,2-octanediol, hydroxyacetophenone, ethylhexylglycerine, hexylene glycol, methyl paraben, propyl paraben, imidazolidinyl urea, sodium dehydroacetate, dimethyl-dimethyl (DMDM) hydantoin and benzyl alcohol and mixtures thereof. Other preservatives include sodium benzoate, sodium dehydroacetate, chlorophenesin and decylene glycol. The preservatives should be selected having regard for the use of the composition and possible incompatibilities between the preservatives and other ingredients in the cleansing composition. Also preferred is a preservative system with hydroxyacetophenone alone or in a mixture with other preservatives. Particularly preferred is sodium benzoate.

The cleansing compositions disclosed herein typically contain water in an amount of 20 to 90% by weight, more particularly 50 to 85% by weight, based on the total weight of the formulation. Such water contents are representative of a relatively broad range of formulations, including both concentrated and non-concentrates products, with formulations having water contents of 20 to less than 50% by weight of water being typical of concentrated products.

In use, the cleansing compositions are commonly diluted with water. The extent of dilution depends on the particular product form. Less commonly, but also contemplated for use herein are formulations that are foamed without dilution, typically through the use of pump dispensers in which product is passed through a screen in the pump.

The cleansing composition can additionally include various additives including, but not limited to, colorants, emollients, anti-dandruff agents, skin feel agents, hair dyes, styling polymer, silicon oil, cationic polymers, or a combination thereof. Each of these substances may range from about 0.03 to about 5%, preferably between 0.1 and 3% by weight of the total weight of the liquid and composition, including all ranges subsumed therein. For example, colorants can be present in an amount of 5 parts per million (ppm) to 15 ppm, for example, about 15 ppm.

Additional optional ingredients which may be present in the subject personal cleansing formulations are, for example: fragrances; sequestering and chelating agents such as tetrasodium ethylenediaminetetraacetate (EDTA), ethane hydroxyl diphosphonate (EHDP), and etidronic acid, aka 1-hydroxyethylidene diphosphonic acid (HEDP); coloring agents; opacifiers and pearlizers such as zinc stearate, magnesium stearate, $TiO_2$, ethylene glycol monostearate (EGMS), ethylene glycol distearate (EGDS) or Lytron 621 (Styrene/Acrylate copolymer); pH adjusters; antioxidants, for example, butylated hydroxytoluene (BHT); stabilizers; suds boosters, such as for example, coconut acyl mono- or diethanol amides; ionizing salts, such as, for example, sodium chloride and sodium sulfate, and other ingredients such as are conventionally used in liquid soap formulations. The total amount of such additional optional ingredients is typically from 0 to 10% by weight, more particularly from 0.1 to 5% by weight, based on the total weight of the personal cleansing formulation.

The compositions typically include one or more skin benefit agents. The term "skin benefit agent" is defined as a substance which softens or improves the elasticity, appearance, and youthfulness of the skin (stratum corneum) by either increasing its water content, adding, or replacing lipids and other skin nutrients, or both, and keeps it soft by retarding the decrease of its water content. Included among the suitable skin benefit agents are emollients, including, for example, hydrophobic emollients, hydrophilic emollients, or blends thereof.

Useful skin benefit agents include the following: (a) silicone oils and modifications thereof such as linear and cyclic polydimethylsiloxanes; amino, alkyl, alkylaryl, and aryl silicone oils; (b) fats and oils including natural fats and oils such as jojoba, soybean, sunflower, rice bran, avocado, almond, olive, sesame, persic, castor, coconut, and mink oils; cacao fat; beef tallow and lard; hardened oils obtained by hydrogenating the aforementioned oils; and synthetic mono, di and triglycerides such as myristic acid glyceride and 2-ethylhexanoic acid glyceride; (c) waxes such as carnauba, spermaceti, beeswax, lanolin, and derivatives thereof; (d) hydrophobic and hydrophilic plant extracts; (e) hydrocarbons such as liquid paraffin, petrolatum, microcrystalline wax, ceresin, squalene, pristan and mineral oil; (f) higher fatty acids such as lauric, myristic, palmitic, stearic, behenic, oleic, linoleic, linolenic, lanolic, isostearic, arachidonic and poly unsaturated fatty acids (PUFA); (g) higher alcohols such as lauryl, cetyl, stearyl, oleyl, behenyl, cholesterol and 2-hexydecanol alcohol; (h) esters such as cetyl octanoate, myristyl lactate, cetyl lactate, isopropyl myristate, myristyl myristate, isopropyl palmitate, isopropyl adipate, butyl stearate, decyl oleate, cholesterol isostearate, glycerol monostearate, glycerol monolaurate, glycerol distearate, glycerol tristearate, alkyl lactate, alkyl citrate and alkyl tartrate; (i) essential oils and extracts thereof such as mentha, jasmine, camphor, white cedar, bitter orange peel, ryu, turpentine, cinnamon, bergamot, citrus unshiu, calamus, pine, lavender, bay, clove, hiba, eucalyptus, lemon, starflower, thyme, peppermint, rose, sage, sesame, ginger, basil, juniper, lemon grass, rosemary, rosewood, avocado, grape, grapeseed, myrrh, cucumber, watercress, calendula, elder flower, geranium, linden blossom, amaranth, seaweed, ginko, ginseng, carrot, guarana, tea tree, jojoba, comfrey, oatmeal, cocoa, neroli, vanilla, green tea, penny royal, aloe vera, menthol, cineole, eugenol, citral, citronelle, borneol, linalool, geraniol, evening primrose, camphor, thymol, spirantol, penene, limonene and terpenoid oils; (j) polyhydric alcohols, for example, glycerine, sorbitol, propylene glycol; and polyols such as the polyethylene glycols, examples of which are: Polyox WSR-205 PEG 14M, Polyox WSR-N-60K PEG 45M, or Polyox WSR-N-750, and PEG 7M; (k) lipids such as cholesterol, ceramides, sucrose esters and pseudo-ceramides as described in European Patent Specification No. 556,957; (l) vitamins, minerals, and skin nutrients such as milk, vitamins A, E, and K; vitamin alkyl esters, including vitamin C alkyl esters; magnesium, calcium, copper, zinc and other metallic components; (m) sunscreens such as octyl methoxyl cinnamate (Parsol MCX) and butyl methoxy benzoylmethane (Parsol 1789); (n) phospholipids; and (o) anti-aging compounds such as alpha-hydroxy acids and beta-hydroxy acids. Skin benefit agents commonly account for up to 30 wt.% of the liquid soap formulation, with levels of from 0 to 25wt.%, more particularly from 0 to 20wt%, being typical of the levels at which those skin benefit agents generally known as "emollients" are employed in many of the subject formulations. Preferred skin benefit agents include fatty acids, hydrocarbons, polyhydric alcohols, polyols and mixtures thereof, with emollients that include at least one $C_{12}$ to $C_{18}$ fatty acid, petrolatum, glycerol, sorbitol and/or propylene glycol being of particular interest in one or more embodiments.

Other optional ingredients include water soluble/dispersible polymers. These polymers can be cationic, anionic, amphoteric, or nonionic types with molecular weights higher than 100,000 Dalton. They are known to increase the viscosity and stability of liquid personal cleansing formulation, to enhance in-use and after-use skin sensory properties, and to enhance lather creaminess and lather stability. When present, the total amount of such polymers commonly ranges from 0.1 to 10% by weight of the personal cleansing formulation.

Examples of water soluble or dispersible polymers include the carbohydrate gums such as cellulose gum, microcrystalline cellulose, cellulose gel, hydroxyethyl cellulose, hydroxypropyl cellulose, sodium carboxymethylcellulose, methyl cellulose, ethyl cellulose, guar gum, gum karaya, gum tragacanth, gum arabic, gum acacia, gum agar, xanthan gum and mixtures thereof; modified and nonmodified starch granules and pregelatinized cold water soluble starch; emulsion polymers such as Aculyn® 28, Aculyn® 22 or Carbopol ®Aqua SF1; cationic polymer such as modified polysaccharides including cationic guar available from Rhone Poulenc under the trade name Jaguar C13S, Jaguar C14S, Jaguar C17, or Jaguar C16; cationic modified cellulose such as UCARE Polymer JR 30 or JR 40 from Amerchol; N-Hance® 3000, N-Hance® 3196, N-Hance® GPX 215 or N-Hance® GPX 196 from Hercules; synthetic cationic polymer such as Merquat® 100, Merquat® 280, Merquat® 281 and Merquat® 550 sold by Nalco; cationic starches such as StaLok® 100, 200, 300 and 400 sold by Staley Inc.; cationic galactomannans such as Galactasol® 800 series by Henkel, Inc.; Quadrosoft® LM-200; and Polyquaternium-24. Also suitable are high molecular weight polyethylene glycols such as Polyox® WSR-205 (PEG 14M), Polyox® WSR-N-60K (PEG 45), and Polyox® WSR-301 (PEG 90M).

A viscosity of the cleansing composition at a shear rate of about 1 s$^{-1}$ can be about 1 Pascal second at a temperature of 25°C. A viscosity of the cleansing composition at a shear rate of about 4 s$^{-1}$ can be about 0.1 Pascal second to about 50 Pascal seconds at a temperature of 25°C.

The cleansing compositions disclosed herein are configured to provide an antimicrobial benefit. The personal cleansing formulations of this invention are of interest with respect to biocidal activity against Gram positive bacteria, including in particular *S. aureus.* Other Gram - positive (Gram-positive) bacteria against which the soap formulations are of interest are *S. epidermidis,* and/or *Corynebacteria,* in particular, *Corynebacteria* strains responsible for the hydrolysis of axilla secretions to malodorous compounds. Desirably, the formulations provides a log$_{10}$ reduction in biocial activity against *Staphylococcus aureus* ATCC 6538 of at least 2.5, preferably of least 3, at a contact time of 30 seconds and even more preferably provides a Log$_{10}$ Reduction against *S. aureus* ATCC 6538 of at least 1.5 even more preferably at least 2 at a contact time of 20 seconds.

The cleansing compositions herein described can be produced by preparative techniques. In one very general methodology, to a heated aqueous phase of water, is added melted fatty acid, followed by caustic (to neutralize the fatty acid and form soap), synthetic detergents and co-solvents; the remaining ingredients are added, as appropriate, as the product is cooled to room temperature.

The cleansing compositions can be provided in a variety of different product forms including, for example, hand, face and body washes, shower gels, bars, e.g., antimicrobial product forms including, for example, hand, face and body washes, shower gels, bars.

The formulations may be provided in bottles, pump dispensers, tubes, sachets, or other packaging suitable for the product form. The cleansing compositions disclosed herein can be used in fatty acid soap/free fatty acid bars that comprise low levels of synthetic surfactant (7 to 25% by weight), high levels of soap and/or high levels of free fatty acid that both process and foam well as described in U.S. Patent No. 6,846,787 to Farrell et al., U.S. Patent No. 6,849,585 to Farrell et al., and U.S. Patent No. 7,351,682 to Moaddel et al.

With the use of a fatty acid/soap stock containing no more than certain amounts of unsaturates and being substantially free of lower chain length fatty acids (e.g., less than C$_{14}$) or fatty acid soaps to make a precursor bar composition before combining with synthetic and other bar components, it is possible to make bars that process and lather well.

Bars can be manufactured by heating a mixer to about 80° to about 90° C, adding the fatty acids, following by addition of caustic to form the precursor, followed by the addition of surfactant and other bar materials. The mixture is dried to a target moisture and then cooled. The cooled material is then extruded, made into billets, and pressed into bars. In use, the cleansing compositions are diluted, as needed, to form aqueous cleansing compositions that are applied to the skin for contact times less than 1 minute, more particularly 30 seconds or less (with contact times of 10 to 30 seconds being of interest with respect to contact times of a moderate to a relatively long duration and contact times of 10 seconds or less being of interest with respect to contact times of short to moderate duration) and thereafter are removed from the skin, typically by rinsing with water. The cleansing compositions can be diluted before, after or simultaneous with its being placed on the skin, with dilution typically occuring by the formulation being worked into a lather in the hands or on an applicator, such as a facecloth, sponge or pouf.

**Figures**

The following is a brief description of the drawings wherein like elements are numbered alike and which are presented for the purposes of illustrating the cleansing compositions disclosed herein and not for the purposes of limiting the same.

FIG. 1 is a graphical illustration of the time-kill study of S. aureus showing the enhanced biocidal action when the surfactant SLES-1 EO is combined with silver ion.
FIG. 2 is a graphical illustration of the viscosity of SLES formulations as a function of salt.

It should be noted that in specifying any range of concentration or amount, any particular upper concentration can be associated with any particular lower concentration or amount as well as any subranges consumed therein. In that regard, it is noted that all ranges disclosed herein are inclusive of the endpoints, and the endpoints are independently combinable with each other (e.g., ranges of "up to 25% by weight, or, more specifically, 5% by weight to 20% by weight, in inclusive of the endpoints and all intermediate values of the ranges of 5% by weight to 25% by weight). "Combination is inclusive of blends, mixtures, alloys, reaction products.

Furthermore, the terms "first", "second", herein do not denote any order, quantity, or importance, but rather are used to distinguish one element from another. The terms "a" and "an" and "the" herein do not denote a limitation of quantity and are to be construed to cover both the singular and the plural, unless otherwise indicated herein or clearly contradicted by context. The suffix "(s)" as used herein is intended to include both the singular and the plural of the term it modifies, thereby including one or more of the term (e.g., the film(s) includes one or more films). Reference throughout the specification to "one embodiment", "one aspect", "another embodiment", "another aspect", "an embodiment", "an aspect" and so forth means that a particular element (e.g., feature, structure, and/or characteristic) described in connection with the embodiment or aspect is included in at least one embodiment or aspect described herein and may or may not be present in other embodiments or aspects. In addition, it is to be understood that the described elements may be combined in any suitable manner in the various embodiments or aspects.

For the avoidance of doubt the word "comprising" is intended to mean "including" but not necessarily "consisting of" or "composed of." In other words, the listed steps, options, or alternatives need not be exhaustive.

The disclosure of the invention as found herein is to be considered to cover all aspects as found in the claims as being multiply dependent upon each other irrespective of the fact that claims may be found without multiple dependency or redundancy.

The Examples provided are to facilitate an understanding of the cleansing composition and are not intended to limit the scope of the claims.

## EXAMPLES

### In-Vitro Time-Kill Protocol

*Surfactant Solution Preparation* - Solution preparation consisted of mixing the surfactant solution with an equal volume of the appropriate buffer to form a test solution containing 50% of the original formulation concentration. Sodium carbonate/bicarbonates buffers (0.1 M) were prepared for use at pH 10 and 7, respectively, while sodium acetate buffer was used at pH 5. Aliquots of silver nitrate solution and salt solution were then added to achieve the desired levels of these components. A series of experiments conducted using dilute NaOH and $HNO_3$ to control the test pH instead of the specified buffers yielded equivalent experimental results but were more tedious.

*Bacteria - Staphylococcus aureus* ATCC 6538 used in this study to represent Gram-positive bacteria was obtained from the American Type Culture Collection (Rockville, Md). The bacteria were stored at -80 °C and fresh working cultures were prepared by streaking on Tryptic Soy Agar plates for 24 hours at 37 °C and then preparing second subcultures in the same way before each experiment.

*In-Vitro Time-Kill Assay-* The Time-Kill assay is performed according to the European Standard, EN 1040:2005 entitled "Chemical Disinfectants and Antiseptics - Quantitative suspension test for evaluation of basic bactericidal activity of chemical disinfectants and antiseptics - Test method and requirements (Phase 1)", with minor modifications as noted. Following this procedure, stationary-phase bacterial cultures at approximately $3 \times 10^8$ colony forming units (CFU) per mL were treated with the surfactant solution (as described above) at 25 °C, rather than at 20 °C as specified in the Standard. In constructing the test sample, 8 parts by weight of the surfactant solution was combined with 1 part by weight of the culture and 1 part by weight of water. After 10, 20, 30, and 60 seconds of exposure, the samples were neutralized to arrest the antibacterial activity of the surfactant solution. These chosen time intervals were shorter than the 5 minutes specified in the Standard so as to be more representative of the time frame consumers typically devote to hand washing. The test solutions were serially diluted, plated on solid medium, and incubated for 24 hours. The surviving cells were then enumerated. Bactericidal activity was defined as the $\log_{10}$ reduction in CFU relative to the initial bacterial concentration at 0 seconds. Cultures not exposed to surfactant solution acted as the no-treatment controls. The $\log_{10}$ reduction was calculated using the formula:

$$\log_{10} \text{ reduction} = \log_{10} (\text{CFU in no-treatment control}) - \log_{10} (\text{CFU in treated sample})$$

**Example 1. SLES/SDS Synergy with Ag over pH range 5-10**

All data gathered were for formulations at 25 °C. All results were the $\log_{10}$ reduction in surviving *S. aureus* after 20/30 seconds exposure from a starting inoculum of about 7.5 $\log_{10}$ and were the average of 3 trials with standard deviation of 0.3 $\log_{10}$ units. The detection limit in all cases was 3 $\log_{10}$.

Table 2

| Sample # | Formulation | $\log_{10}$ survivor (CFU/mL) 20/30 s | log reduction @ 20 s | log reduction @ 30 s |
|---|---|---|---|---|
| 1 | **pH 10 control** | 7.4/7.4 | ---- | ----- |
| 2 | 10% SLES-1EO* at pH 10 | 7.0/7.0 | 0.4 | 0.4 |
| 3 | 10% SLES-1EO + 0.5 ppm Ag$^+$ | 3.3/3.3 | 4.1 | 4.1 |
| 4 | 10% Na Decanoate | 7.3/7.3 | 0.1 | 0.1 |
| 5 | 10% Na Decanoate + 1 ppm Ag$^+$ | 3.3/3.3 | 4.1 | 4.1 |
| 6 | 5% SLES-1EO/5% NaC$_{10}$ | 7.3/7.3 | 0.1 | 0.1 |
| 7 | SLES-1EO/NaC$_{10}$ + 0.3 ppm Ag$^+$ | 3.3/3.3 | 4.1 | 4.1 |
| 8 | 5% SLES-3EO**/5% NaC$_{10}$ | 7.4/7.4 | 0.0 | 0.0 |
| 9 | SLES-3EO/NaC$_{10}$ + 0.3 ppm Ag$^+$ | 3.3/3.3 | 4.1 | 4.1 |
| | | | | |
| 10 | **pH 7 control** | 7.5/7.5 | ---- | |
| 11 | 10% SLES-1EO at pH 7 | 7.0/6.6 | 0.5 | 0.9 |
| 12 | 10% SLES + 0.5 ppm Ag$^+$ at pH 7 | 3.7/3.3 | 3.8 | 4.2 |
| | | | | |
| 13 | **pH 5 control** | 7.5/7.4 | ---- | ---- |
| 14 | 10% SLES-1EO at pH 5 | 5.6/5.2 | 1.9 | 2.2 |
| 15 | 10% SLES + 0.3 ppm Ag$^+$ at pH 5 | 3.9/3.3 | 3.6 | 4.1 |
| 16 | 10% SLES + 0.5 ppm Ag$^+$ at pH 5 | 4.4/3.6 | 3.1 | 3.8 |
| 17 | 10% SLES + 1 ppm Ag$^+$ at pH 5 | 3.3/3.3 | 4.2 | 4.1 |
| *SLES-1EO refers to sodium lauryl ether sulfate with 1 ethoxy group ** SLES-3EO refers to sodium lauryl ether sulfate with 3 ethoxy groups | | | | |

This example demonstrated the wide pH range over which the biocidal activity of surfactant/silver formulations was active. The sulfate surfactant SLES-1 EO gave a $\log_{10}$ reduction of about 0.5 units in the pH range of 7-10, but this reduction was markedly increased to about 4 units in the presence of silver ion. At pH 5, SLES was biocidal on its own with a $\log_{10}$ reduction of about 2. Even in this case, there was room for an additional 1 to 2 log reduction upon addition of silver.

A similar level of benefit was observed at pH 10 for sodium decanoate soap and for 50/50 blends of this soap with SLES-1 EO or with SLES-3EO. Soap was not an appropriate surfactant for use in this test in the pH range 5-7 due to the insolubility of the corresponding fatty acids.

It can be concluded that sulfate surfactants combined with silver ion in the spirit of the cleansing compositions disclosed herein showed an enhanced biocidal action over the pH range of 5-10. Further, soap surfactants and soaps combined with sulfate surfactants also showed this enhanced biocidal action at a soap appropriate pH. At the lower pH limit, these formulations will be compatible with the use of organic acids as preservatives. For instance, the percentage of organic acid preservative remaining undissociated (and active) at pH 5 is 13% for benzoic acid and 37% for sorbic acid (G. Hill, "Preservation of cosmetics and toiletries", in Handbook of Bioicides and Preservatives Use, edited by H.W. Rossmoore, Springer Science and Business, 1995).

## Example 2. Uniqueness of Sulfates

In Example 2, surfactants typically used in personal washing were tested for synergy with silver ion at pH 5 and pH 7.

All results in Table 3 were recorded as the $\log_{10}$ reduction in surviving *S. aureus* after 20/30 seconds exposure at pH 5 from a starting inoculum of about 7.5 $\log_{10}$ and were the average of 3 trials with standard deviation of 0.3 $\log_{10}$ units. The detection limit in all cases was 3 $\log_{10}$.

Table 3

| Sample # | Formulation | $\log_{10}$ survivor CFUlmL) 20/30 s | log reduction @ 20 s | log reduction @ 30 s |
|---|---|---|---|---|
| 18 | **pH 5 control** (acetate buffer) | 7.4 | ---- | ---- |
| 19 | 10% Triton X-100 | 7.4 | 0.0 | 0.0 |
| 20 | 10% Triton + 1 ppm Ag$^+$ | 7.4 | 0.0 | 0.0 |
| 21 | 10% Na Methyl Cocoyl Taurate | 7.4 | 0.0 | 0.0 |
| 22 | 10% Taurate + 1 ppm Ag$^+$ | 7.4 | 0.0 | 0.0 |
| 23 | 10% SLES-1EO | 6.7/6.6 | 0.7 | 0.8 |
| 24 | 10% SLES + 0.5 ppm Ag$^+$ | 4.2/3.5 | 3.2 | 3.9 |
| 25 | 10% SLES + 1 ppm Ag$^+$ | 3.3/3.3 | 4.1 | 4.1 |
| 26 | 10% SDS | 3.3 | 4.1 | 4.1 |
| 27 | 10% SDS + 1 ppm Ag$^+$ | 3.3 | 4.1 | 4.1 |

Table 3, with all examples at pH 5, demonstrated that most of the surfactants suitable for personal washing applications and which were capable of dissolving at this pH were not biocidal - either inherently or in combination with silver ion. Examples of such ineffective surfactants include nonionic surfactants such as Triton X-100 (octyl phenol ethoxylate). This commercially available detergent is widely used to lyse cells for the purpose of extracting proteins or organelles and to permeabilize cell membranes ("Triton X-100 concentration effects on membrane permeability of a single HeLa cell by scanning electrochemical microscopy (SECM)", by D. Koley and A.J. Bard, PNAS 107, 16783-16787 (2010); "Classifying surfactants with respect to their effect on lipid membrane order", M. Nazari et al., Biophysical Journal 102, 498-506 (2012)). Concentrations of the detergent above its CMC (Critical Micelle Concentration) (= 0.015%) disrupt the hydrogen bonding within a cell's lipid bilayer and destroy the integrity of the lipid membrane. This situation can be lethal to cells at an exposure time on the order of minutes. Concentrations slightly below the CMC permeabilized the cell membrane sufficiently to allow highly charged, hydrophilic ions (which would normally be completely excluded) to pass through the living cell membrane. Despite these properties, Triton X-100 was not biocidal, even at the elevated level of 10 wt.%, on the 10-60 second time scale appropriate to handwashing and apparently does not facilitate the penetration of silver ion in the *S. aureus* cell.

Sodium methyl cocoyl taurate is the sodium salt of the coconut fatty acid amide of N-methyltaurine. It is classified as a sulfonate rather than a sulfate detergent and is soluble over a wide pH range, showing the typical surfactant properties of foaming and surface tension lowering. However, it does not have intrinsic biocidal properties, nor does it enhance the biocidal action of silver ion at pH 5.

Conversely, the sulfates SDS and SLES show strong synergy with silver ion at pH 5. (though SDS is quite biocidal on its own at this pH).

Table 4 shows examples of the synergistic biocidal action of surfactants typically used in personal washing applications at pH 7 with silver. All results were the $\log_{10}$ reduction in surviving *S. aureus* after 20/30 seconds exposure from a starting inoculum of about 7.5 $\log_{10}$ and were calculated as the average of 3 trials with standard deviation of 0.3 $\log_{10}$ units. The detection limit in all cases is 3 $\log_{10}$.

Table 4

| Sample # | Formulation | $\log_{10}$ survivor CFU/mL) 20/30 s | log reduction @ 20 s | log reduction @ 30 s |
|---|---|---|---|---|
| 28 | **pH 7 control** (carbonate buffer) | 7.5 | ---- | ---- |
| 29 | 10% Triton X-100 | 7.5/7.4 | 0.0 | 0.1 |
| 30 | 10% Triton + 1 ppm Ag$^+$ | 7.4/7.4 | 0.1 | 0.1 |
| 31 | 10% Taurate | 7.5/7.5 | 0.0 | 0.0 |
| 32 | 10% Taurate + 0.5 ppm Ag$^+$ | 7.25/6.9 | 0.25 | 0.6 |
| 33 | 10% Taurate + 1 ppm Ag$^+$ | 6.2/5.4 | 1.3 | 2.1 |
| 34 | 5% Na Cocoyl Glycinate | 7.5 | 0.0 | 0.0 |
| 35 | 5% Glycinate + 1 ppm Ag$^+$ | 7.5 | 0.0 | 0.0 |
| 36 | 10% Cocoyl Glutamate | 7.5 | 0.0 | 0.0 |
| 37 | 10% Glutamate + 0.4 ppm Ag$^+$ | 7.4 | 0.1 | 0.1 |
| 38 | 10% Glutamate + 1 ppm Ag$^+$ | 7.4 | 0.1 | 0.1 |
| 39 | 10% Amphoacetate | 7.5 | 0.0 | 0.0 |
| 40 | 10% Amphoacetate+0.4 ppm Ag$^+$ | 7.5 | 0.0 | 0.0 |
| 41 | 10% Amphoacetate + 1 ppm Ag$^+$ | 7.5 | 0.0 | 0.0 |
| 42 | 10% SLES-1EO | 6.9/6.7 | 0.6 | 0.8 |
| 43 | 10% SLES-1EO + 0.5 ppm Ag+ | 3.3 | 4.2 | 4.2 |
| 44 | 10% SLES-3EO | 7.5 | 0.0 | 0.0 |
| 45 | 10% SLES-3EO + 0.5 ppm Ag+ | 3.3 | 4.2 | 4.2 |
| 46 | 10% SDS | 5.3/5.1 | 2.2 | 2.4 |
| 47 | 10% SDS + 0.5-1 ppm Ag+ | 3.3 | 4.2 | 4.2 |

Table 4, with all samples at pH 7, demonstrated that most of the surfactants suitable for personal washing applications and which were capable of dissolving at this pH were not biocidal - either inherently or in combination with silver ion. Examples of such ineffective surfactants again include the nonionic surfactants such as Triton X-100 and the sulfonate detergent sodium methyl cocoyl taurate. Additional ineffective surfactants include the anionic amino-acid based surfactants sodium cocoyl glycinate, sodium cocoyl glutamate, and the amphoteric surfactant sodium lauroamphoacetate. These ineffective surfactants could contain considerable levels of NaCl as an impurity left over from their manufacture (up to 20% salt by weight of solids - "Sodium Lauroamphoacetate" M.J. Fevola, Cosmetics & Toiletries 126, 844-848 (2011).), which would be expected to render the added silver ion ineffective. To eliminate this complication, the surfactants were all treated by repeated recrystallization from cold water until their chloride ion concentration was below the 10 ppm detection limit by ion chromatography.

Throughout the pH interval claimed, the sulfated fatty alcohols (SDS) and sulfated polyoxyethylated alcohols (SLES-1EO and SLES-3EO) have limited inherent biocidal action but showed strong synergy with silver ion.

**Example 3. Effect of Chloride Ion on Biocidal Activity of Surfactant/Silver Formulations**

Chloride ion is present in natural waters as a result of dissolved minerals such as NaCl, KCl, and $CaCl_2$, or as due to the use of chloride salts for snow and ice control ("Guidelines for drinking-water quality", 2nd ed. Vol. 2. Health criteria and other supporting information, World Health Organization, Geneva, 1996). The mean chloride ion concentration in UK rivers was found to range from 11 to 42 milligrams per Liter mg/L ($3 \times 10^{-4}$ to $1.2 \times 10^{-3}$ M), while aquifers in the USA can contain over 100 mg/L ($2.8 \times 10^{-3}$ M) chloride ion. In addition, chloride ion is present in personal washing products, either as an impurity or as a deliberate additive. Secondary surfactants are frequently added to such products to boost their skin compatibility and to optimize their overall performance in terms of cleaning and sudsing (S. Herrwerth et al., "Highly concentrated Cocamidopropyl betaine - The latest developments for improved sustainability and enhanced skin care", Tenside 45, 304-308 (2008)). The most important class of secondary surfactants are the amidopropyl betaines, which are produced in a two-step process, the second step of which involves reaction with chloroacetic acid and results in the formation of sodium chloride as a by-product. In fact, the sodium chloride levels of commercially available betaines can range from 4-9%, so that even when these secondary surfactants are used at levels of a few percent in a product, they contribute on the order of 0.03 M chloride ion. In addition, chloride salts such as NaCl are often added to liquid personal washing products to boost their viscosity. Addition of 1-2% NaCl is typical, which contributes an additional 0.1-0.3 M chloride ion.

This example illustrated how chloride ion has a deleterious effect on the biocidal properties of silver-containing formulations because of the low solubility of silver chloride.

Table 5 shows all data for formulations at pH 7.0 and at 25 °C. All results were reported as the $\log_{10}$ reduction in surviving *S. aureus* after 20/30 seconds exposure from a starting inoculum of about 7.5 $\log_{10}$ and were calculated as the average of 3 trials with standard deviation of 0.3 $\log_{10}$ units. The detection limit in all cases was 3 $\log_{10}$.

Table 5

| Sample # | Formulation | $\log_{10}$ survivor (CFU/mL) 20/30 s | log survivor @ 20 s | log survivor @ 30 s |
|---|---|---|---|---|
| 48 | **pH 7 control** | 7.4 | ---- | ---- |
| 49 | 1 or 10 ppm Ag+ | 7.4 | 0.0 | 0.0 |
| 50 | 1% SLES-1 EO | 6.8/6.7 | 0.6 | 0.7 |
| 51 | 1% SLES + 1 ppm Ag$^+$ | 3.3 | 4.1 | 4.1 |
| 52 | 1% SLES + 10 ppm Ag$^+$ | 3.3 | 4.1 | 4.1 |
| 53 | 1% SLES/1 ppm Ag$^+$ + 1 M NaCl | 4.7/4.0 | 2.7 | 3.4 |
| | | | | |
| 54 | 1 ppm Ag$^+$ | 7.4 | 0.0 | 0.0 |
| 55 | 10% SLES-1EO | 7.1/7.0 | 0.3 | 0.4 |
| 56 | 10% SLES + 0.17 M NaCl | 6.8/6.7 | 0.6 | 0.7 |
| 57 | 10% SLES + 0.5 M NaCl | 6.7/6.5 | 0.7 | 0.9 |
| 58 | 10% SLES + 0.3 ppm Ag$^+$ | 3.3 | 4.1 | 4.1 |
| 59 | 10% SLES + 1 ppm Ag$^+$ | 3.3 | 4.1 | 4.1 |
| 60 | SLES/0.3 ppm Ag$^+$ + 0.5 M NaCl | 5.4/5.1 | 2.0 | 2.3 |
| 61 | SLES/1 ppm Ag$^+$ + 0.03 M NaCl | 6.5/6.0 | 0.9 | 1.4 |
| 62 | SLES/1 ppm Ag$^+$ + 0.17 M NaCl | 6.7/5.9 | 0.7 | 1.5 |
| 63 | SLES/1 ppm Ag$^+$+ 0.5 M NaCl | 5.6/5.0 | 1.8 | 2.4 |
| 64 | SLES/3 ppm Ag$^+$+ 0.5 M NaCl | 5.0/4.3 | 2.4 | 3.1 |

In the top-half of Table 5, 1 to 10 ppm Ag$^+$ alone at pH 7 had no biocidal effect. The surfactant SLES at 1% gave a weak biocidal effect of $\leq$ 1 log$_{10}$ unit reduction. The combination of SLES and silver yielded a strong biocidal effect (4 log$_{10}$ reduction), but this effect was compromised in the presence of NaCl.

The second half of Table 5, pertaining to a higher surfactant level of 10% SLES, displays the following results: 1 ppm Ag$^+$ alone at pH 7 had no biocidal effect. The surfactant SLES at 10% gave a weak biocidal effect of $\leq$ 1 log$_{10}$ unit reduction which is not significantly improved by the addition of up to 0.5 M NaCl. The combination of SLES and silver yielded a strong biocidal effect (4 log$_{10}$ reduction), but this effect was compromised in the presence of NaCl. Increasing the level of silver ion from 1 to 3 ppm was ineffective in restoring the biocidal action when NaCl was present at levels up to 0.5 M.

In summary, the level of surfactant ranging from 1 to 10% was not intrinsically biocidal but became so in the presence of ppm levels of silver ion. However, the biocidal activity was greatly reduced in the presence of high levels of chloride ion such as are commonly found in personal washing products (> 0.01 M).

## Example 4. Effect of salt on viscosity

The addition of salts to liquid personal wash products as a means of increasing the product's viscosity is a very common procedure. Consumers have come to expect a certain level of product thickness which they associate with the richness and effectiveness of the product. Water-thin products give the impression of being unsubstantial and of inferior quality.

It is broadly understood that the effect of salts such as NaCl on viscosity is linked to their role in increasing the size of the micelles formed by the detersive surfactants (B. Jonsson et al., "Surfactants and Polymers in Aqueous Solution", John Wiley & Sons, 1998). The effect of NaCl on micelle size in a series of sodium alky sulfates has been studied by R. Ranganathan et al., "Surfactant- and salt-induced growth of normal sodium alkyl sulfate micelles well above their critical micelle concentrations", J. Phys. Chem 8 104, 2260-2264 (2000), with the finding that micelle size showed power law growth with added NaCl salt in the range of 0.1-1 M. For this reason, it is desirable to add NaCl to skin cleansing formulations and it is a particular disadvantage that such additions greatly reduce the biocidal enhancement seen when surfactant and silver are combined.

Table 6 shows the viscosity of 10% SLES-1 EO formulations as a function of salt level. Measurements are made using a TA Instruments Discovery HR-2 Hybrid Rheometer with a 4 cm flat-plate geometry and 1000 $\mu$m gap at a temperature of 25°C. The measuring surfaces are sandblasted to avoid wall slip and the results are reported for a shear rate of 4 s$^{-1}$.

Table 6

| Sample # | NaCl level, M | $Log_{10}(\eta, Pa\,s)$ | NaNO$_3$ level, M | $Log_{10}(\eta, Pa\,s)$ |
|---|---|---|---|---|
| 65 | 0.1 | -2.54 | 0.1 | -2.59 |
| 66 | 0.2 | -2.32 | 0.2 | -2.51 |
| 67 | 0.3 | -1.88 | 0.3 | -1.95 |
| 68 | 0.4 | -0.73 | 0.4 | -0.77 |
| 69 | 0.5 | 0.19 | 0.5 | -0.26 |
| 70 | 0.6 | 0.89 | 0.6 | 0.59 |
| 71 | 0.7 | 1.22 | 0.7 | 1.01 |
| 72 | 0.8 | 1.39 | 0.8 | 1.21 |
| 73 | 1.0 | 1.38 | 1.0 | 1.40 |

Table 6 and Figure 2 demonstrated the strong viscosity enhancement achieved by adding 0.1-1 M NaCl. The viscosity was reported at a shear rate of 4 s$^-$ at a temperature of 25°C $^1$, which was a deformation rate traditionally used to assess skin cleansing formulations. A very similar degree of enhancement can be achieved with the nitrate analog to NaCl, NaNO$_3$. It is also noteworthy that the sodium nitrate levels which gave a strong viscosity enhancement (0.4 M and up) were the same ones necessary to recover the synergistic action between surfactant and silver lost in the presence of impurity levels of NaCl (see Example 6).

**Example 5. Effect of Polymers**

Polymers having a repeat unit containing one or more lone pairs of electrons have been claimed (US 2016/0362646 A1) to have a strong affinity for silver ions and so reduce their tendency to agglomerate. As a result, the antimicrobial efficacy of the metal ion is enhanced. A preferred embodiment of this class of polymers is polyvinylpyrrolidone employed at 0.002 to 2% of the total composition. V. Sambhy, M.M. MacBride, B.R. Peterson and A. Sen, "Silver bromide nanoparticle/polymer composites: Dual action tunable antimicrobial materials", J. Am. Chem. Soc. 128, 9798-9808 (2006) report that poly(4-vinylpyridine) copolymers act as stabilizers for silver. In addition, gelatin has also been utilized for many years in the photography industry as a stabilizer for silver colloids, preventing them from agglomerating (M. Yang, J.-G. Zhao, J.-J. Li, "Synthesis of porous spherical AgBr nanoparticles in the presence of gelatin using AgCl as the precursor", Colloids and Surfaces A 295, 81-84 (2007).

A separate claim is made in US 2011/0224120 A1 for non-neutralized polycarboxylic acids used between pH 7.5 and 8.5 at levels of 0.01 to 10 wt% and in US 2012/0034314 A1 for silver chelated by polyacrylate polymer at levels of 0.1-2% and at pH 6-9. These polycarboxylates are claimed to act as stabilizers for silver ion, preventing precipitation and color change *without* affecting biocidal action. A further benefit of polycarboxylic acids is that they are often added to personal washing products as viscosity enhancers, to provide the look and substantial feel that consumers have come to expect from such products.

The example below demonstrated that the above quoted examples actually teach away from the presently claimed composition.

Table 7 shows the effect of polymers which interact with silver ions on the synergistic biocidal action of surfactant and silver. PVPi is poly (4-vinylpyridine), PVPo is polyvinylpyrrolidone, PAAn is polyallylamine, PEI is poly ethyleneimine. All results were the $log_{10}$ reduction in surviving *S. aureus* after 20/30 seconds exposure at 25 °C from a starting inoculum of about 7.5 $log_{10}$ and were the average of 3 trials with standard deviation of 0.3 $log_{10}$ units. The detection limit in all cases is 3 $log_{10}$.

Table 7

| Sample # | Formulation | $log_{10}$ survivor CFUlmL) 20/30 s | log reduction @ 20 s | log reduction @ 30s |
|---|---|---|---|---|
| 65 | **pH 7 control** | 7.3/7.4 | ---- | ---- |
| 66 | 10% SLES | 7.0/6.9 | 0.3 | 0.5 |
| 67 | SLES + 1 ppm Ag$^+$ | 3.3 | 4.0 | 4.1 |
| 68 | SLES/Ag$^+$ + 0.03 M NaCl | 5.8/4.5 | 1.5 | 2.9 |
| 69 | SLES/Ag$^+$/NaCl + 0.1% Gelatin | 6.1/4.7 | 1.2 | 2.7 |
| 70 | SLES/Ag$^+$/NaCl + 0.1% PVPi | 5.2/3.8 | 2.1 | 3.6 |
| | | | | |
| 71 | SLES + 0.4 ppm Ag$^+$ | 3.3 | 4.0 | 4.1 |
| 72 | SLES + 0.4 ppm Ag$^+$+ 0.1 M NaCl | 6.2/5.75 | 1.1 | 1.6 |
| 73 | SLES/Ag$^+$/NaCl + 0.1% PVPo | 6.4/6.0 | 0.9 | 1.4 |
| 74 | SLES/Ag$^+$/NaCl + 0.1% PAAn | 5.9/5.4 | 1.4 | 2.0 |
| 75 | SLES/Ag$^+$/NaCl + 0.1% PEI | 6.7/6.6 | 0.6 | 0.8 |

Table 7 demonstrated that addition of gelatin, poly(4-vinylpyridine), polyvinylpyrrolidone, polyallylamine, or polyethyleneimine did not adequately protect the SLES/Ag system from loss of biocidal activity in the presence of 0.03-0.1 M NaCl. Thus PVPi, PVPo, PAAn, and PEI, all polymers with lone pairs of electrons as claimed in US 2016/0362646 A1 to have a strong affinity for silver ions, did not protect the SLES/Ag system from the deleterious effect of NaCl. Accordingly, US 2016/0362646 therefore teaches away from the present cleansing composition.

Table 8 shows the effect of polyacrylates which interact with silver ions on the synergistic biocidal action of surfactant and silver. All results were the $log_{10}$ reduction in surviving *S. aureus* after 20/30 seconds exposure at 25 °C from a starting inoculum of about 7.5 $log_{10}$ and were the average of 3 trials with standard deviation of 0.3 $log_{10}$ units.

The detection limit in all cases was 3 $\log_{10}$.

Table 8

| Sample # | Formulation | $\log_{10}$ survivor (CFU/mL) 20/30 s | $\log_{10}$ reduction @ 20 s | $\log_{10}$ reduction @ 30 s |
|---|---|---|---|---|
| 76 | **pH 7 bicarbonate buffer** | 7.3/7.2 | ---- | ---- |
| 77 | 10% SLES + 0.2 ppm Ag$^+$ | 3.3/3.3 | 4.0 | 3.9 |
| 78 | 10% SLES + 0.4 ppm Ag$^+$ | 3.3/3.3 | 4.0 | 3.9 |
| 79 | 10% SLES + 1 ppm Ag$^+$ | 3.3/3.3 | 4.0 | 3.9 |
| | | | | |
| 80 | SLES/0.2 ppm Ag$^+$ + 1% PAA | 5.5/5.0 | 1.8 | 2.2 |
| 81 | SLES/0.4 ppm Ag$^+$+ 1% PAA | 4.6/4.0 | 2.7 | 3.2 |
| 82 | SLES/1 ppm Ag$^+$ + 1% PAA | 3.8/3.6 | 3.5 | 3.6 |
| | | | | |
| 83 | SLES/0.4 ppm Ag$^+$ | 3.3/3.3 | 4.0 | 3.9 |
| 84 | SLES/Ag$^+$ + 0.1% PAA | 3.8/3.6 | 3.5 | 3.6 |
| 85 | SLES/Ag+/0.03 M NaCl | 5.5/4.8 | 1.8 | 2.4 |
| 86 | SLES/Ag+/0.03 M NaCl + 0.1% PAA | 5.8/5.2 | 1.5 | 2.0 |
| 87 | SLES/Ag+ + 0.1 M NaCl | 6.2/6.0 | 1.1 | 1.2 |
| 88 | SLES/Ag+/NaCl + 0.1% PAA | 6.4/6.2 | 0.9 | 1.0 |

Table 8 demonstrated that while the addition of 0.2 - 1 ppm of silver ion yielded synergistic biocidal action against *S. aureus,* 1% polyacrylic acid markedly reduced the synergy between the surfactant and 0.2 ppm silver, a finding that could not have been predicted from reference to US 2011/0224120 A1 for non-neutralized polycarboxylic acids used between pH 7.5 and 8.5 at levels of 0.01 to 10% by weight. In this reference, polycarboxylates are claimed to act as stabilizers for silver ion, preventing precipitation and color change without affecting biocidal action. Similarly, US 2012/0034314A claims that silver ion associated with polyacrylate polymer at levels of 0.1-2% and at a pH of 6-9 maintains a stable, long-lasting antimicrobial benefit. Rows 6 and 7 of Table 8 (Samples 81 and 82) demonstrate that some level of synergy reduction is present at 0.4 and 1 ppm Ag$^+$ as well. Rather than stabilizing silver ion without interfering with biocidal action, it would appear instead that the polycarboxylate was complexing the silver ion. Evidence for this complexation was the fact that the synergy improved as the total silver level was increased from 0.2 to 1 ppm at fixed polycarboxylate level.

Rows 8-13 of Table 8 (Samples 83-88) explored the possibility that polycarboxyates could interfere with precipitation of silver chloride and hence protect the biocidal enhancement of silver by surfactant in the presence of added NaCl, as suggested by US 2011/0224120 A1. The addition of 0.03 and 0.1M NaCl progressively reduces the enhancement by surfactant due to precipitation of AgCl. But addition of 0.1% polycarboxylates, rather than improving the situation, makes it marginally worse, so that there is no indication that these polymers act as crystallization inhibitors either.

**Example 6. Benefits of nitrate salts**

Natural waters can contain a wide range of sodium chloride. Fresh water contains nearly zero salt, while the average salinity in the world's oceans is about 35 parts per thousand (3.5% or 0.6 M) (https://ww2.health.wa.gov.au/Articles/S_T/Sodium-in-drinking-water). Seawater and fresh water often mix in estuaries and river mouths, yielding brackish water whose salinities can range upwards of 0.5 parts per thousand. The Department of Health in Australia has determined that salt levels in excess of 0.18 parts per thousand (0.018% or 3 mM) will give water a salty taste. Yet such waters, while unsuitable for drinking, could still be used for personal washing. Indeed, the use of such non-potable water for washing, if feasible, would be preferable to "wasting" fresh water which could be in short supply.

Chloride ions in water, which typically enter as a result of dissolved sodium chloride, can have a deleterious effect on the antibacterial action of silver. The second line in Table 9 (Sample 90) displays the excellent biocidal activity of the formulation against S. aureus at 20/30 seconds exposure, with a 4 $log_{10}$ reduction in viable organisms. The third line (Sample 91) illustrates the effect of adding 0.5 M NaCl to an antibacterial formulation (20% SLES and 0.3 ppm $Ag^+$) in the suspension test. Addition of NaCl significantly eliminates the biocidal action and it is not possible to recover the activity by adding excess silver ion.

Table 9 shows the effect of changing the electrolyte from NaCl to $NaNO_3$ on the enhancement of biocidal action upon combining surfactant and silver. All data were for formulations at pH 7.0 and at 25 °C. All results were the $log_{10}$ reduction in surviving S. *aureus* after 20/30 seconds exposure from a starting inoculum of about 7.5 $log_{10}$ and were the average of 3 trials with standard deviation of 0.3 $log_{10}$ units. The detection limit in all cases was 3 $log_{10}$.

Table 9

| Sample # | Formulation | $log_{10}$ survivor (CFU/mL) 20/30 s | log reduction @ 20s | log reduction @ 30 s |
|---|---|---|---|---|
| 89 | **pH 7 control** | 7.3 | ---- | ---- |
| 90 | 20% SLES, 0.3 ppm $Ag^+$ | 3.3 | 4.0 | 4.0 |
| 91 | 20% SLES/0.5 M NaCl, 0.3 ppm $Ag^+$ | 5.4/5.1 | 1.9 | 2.2 |
| 92 | 20% SLES/0.5 M NaCl, 1 ppm $Ag^+$ | 5.4/4.8 | 1.9 | 2.5 |
| 93 | 20% SLES/0.5 M NaCl, 3 ppm $Ag^+$ | 5.0/4.3 | 2.3 | 3.0 |
|  |  |  |  |  |
| 94 | 20% SLES/0.5 M $NaNO_3$, 0.3 ppm $Ag^+$ | 3.3 | 4.0 | 4.0 |
| 95 | 20% SLES/0.5 M $NaNO_3$, 1 ppm $Ag^+$ | 3.3 | 4.0 | 4.0 |
| 96 | 20% SLES/0.5 M $NaNO_3$, 3 ppm $Ag^+$ | 3.3 | 4.0 | 4.0 |

Lines 6-8 of Table 9 (Samples 94-96) demonstrate that while the addition of NaCl largely eliminates the biocidal action, substitution of sodium nitrate for sodium chloride as electrolyte completely restores it. As sodium nitrate showed a similar thickening tendency with SLES as does sodium chloride, it can be used as a thickening agent without sacrificing biocidal action.

Table 10 shows all data for formulations at pH 7.0 and at 25 °C. All results were the $log_{10}$ reduction in surviving S. *aureus* after 20/30 seconds exposure from a starting inoculum of about 7.5 $log_{10}$ and were the average of 3 trials with standard deviation of 0.3 $log_{10}$ units. The detection limit in all cases was 3 $log_{10}$.

Table 10

| Sample # | Formulation | $log_{10}$ survivor (CFU/mL) 20/30 s | log reduction @ 20 s | log reduction @ 30 s |
|---|---|---|---|---|
| 97 | **pH 7 control** | 7.3 | ---- | ---- |
| 98 | 10% SLES + 0.4 ppm $Ag^+$ | 3.6/3.3 | 3.7 | 4.0 |
| 99 | SLES/$Ag^+$ + 0.03 M NaCl | 6.4/6.0 | 0.9 | 1.3 |
| 100 | SLES/$Ag^+$ /NaCl + 0.05 M $NaNO_3$ | 6.1/5.7 | 1.2 | 1.6 |
| 101 | SLES/$Ag^+$ /NaCl + 0.1 M $NaNO_3$ | 6.1/5.6 | 1.2 | 1.7 |
| 102 | SLES/$Ag^+$ /NaCl + 0.3 M $NaNO_3$ | 5.7/5.3 | 1.6 | 2.0 |
| 133 | SLES/$Ag^+$/NaCl + 0.5 M $NaNO_3$ | 5.4/4.6 | 1.9 | 2.7 |
| 104 |  |  |  |  |
| 105 | SLES/0.4 ppm $Ag^+$/0.1 M NaCl | 5.9/5.5 | 1.4 | 1.8 |
| 106 | SLES/0.4 $Ag^+$/0.1 NaCl + 0.5 M $NaNO_3$ | 4.5/4.1 | 2.8 | 3.2 |

(continued)

| Sample # | Formulation | $log_{10}$ survivor (CFU/mL) 20/30 s | log reduction @ 20 s | log reduction @ 30 s |
|---|---|---|---|---|
| 107 | | | | |
| 108 | SLES/1 ppm $Ag^+$/0.03 M NaCl | 5.8/4.8 | 1.5 | 2.5 |
| 109 | SLES/1 $Ag^+$/0.03 NaCl + 0.5 M $NaNO_3$ | 4.0/3.3 | 3.3 | 4.0 |

Again Table 10 demonstrates that the synergistic biocidal action found when combining surfactant and silver is severely compromised by as little as 0.03 M NaCl. These levels of NaCl can be present in the formulation as an impurity of the surfactant, added for purposes of increasing formulation viscosity, or can enter the wash liquor via a brackish water supply. It can be concluded that 0.3 M or higher $NaNO_3$ is necessary to recover a one unit $log_{10}$ reduction in *S. aureus* in the presence of 0.03 M NaCl (0.175% NaCl). As this salt level is ten times the level at which water tastes salty, it is clear that even such non-potable waters could still be used for biocidal cleansing upon consideration of this invention.

**Example 7. Silver ion requirement for surfactant/silver synergy in presence of sodium nitrate**

In Table 11, all data for formulations was at pH 7.0 and at 25 °C. All results were the $log_{10}$ reduction in surviving *S. aureus* after 20/30 seconds exposure from a starting inoculum of about 7.5 $log_{10}$ and were the average of 3 trials with standard deviation of 0.3 $log_{10}$ units. The detection limit in all cases was 3 $log_{10}$.

Table 11

| Sample # | Formulation | $log_{10}$ survivor (CFU/mL) 20/30 s | log reduction @ 20 s | log reduction @ 30 s |
|---|---|---|---|---|
| 110 | **pH 7 control** | 7.4 | ---- | ---- |
| 111 | 10% SLES + 0.5 M $NaNO_3$ | 6.8/6.5 | 0.6 | 0.9 |
| 112 | SLES/$NaNO_3$ + 3 ppm $Ag^+$ | 3.3 | 4.1 | 4.1 |
| 113 | SLES/$NaNO_3$ + 1 ppm $Ag^+$ | 3.3 | 4.1 | 4.1 |
| 114 | SLES/$NaNO_3$ + 0.3 ppm $Ag^+$ | 3.3 | 4.1 | 4.1 |
| 115 | SLES/$NaNO_3$ + 0.1 ppm $Ag^+$ | 3.3 | 4.1 | 4.1 |
| 116 | SLES/$NaNO_3$ + 0.03 ppm $Ag^+$ | 3.3 | 4.1 | 4.1 |
| 117 | SLES/$NaNO_3$ + 0.01 ppm $Ag^+$ | 5.2/4.7 | 2.2 | 2.7 |

This example demonstrated that the minimum level of silver ion necessary to see enhanced biocidal action in the presence of 10% SLES and 0.5 M $NaNO_3$ was $\geq$ 0.03 ppm under the conditions of pH 7 and 25 °C.

**Example 8. Surfactant/silver/nitrate system more tolerant of polycarboxylates**

Polycarboxylates are claimed to act as stabilizers for silver ion, preventing precipitation and color change without affecting biocidal action. A further benefit of polycarboxylic acids is that they are often added to personal washing products as viscosity enhancers, to provide the look and substantial feel that consumers have come to expect from such products.
The example below demonstrates that polycarboxylic acids interfere with the biocidal action of surfactant plus silver, teaching away from the prior disclosed cleansing compositions. However, biocidal action in the surfactant/silver/nitrate system is more tolerant of polycarboxylates than in the absence of nitrate and so is more amenable to the addition of polycarboxylates for viscosity control.
Table 12 shows the effect of polycarboxylates which interact with silver ions on the synergistic biocidal action of surfactant and silver. All results were an average of 3 trials with standard deviation of 0.5 $\log_{10}$ units. The detection limit in all cases was 3 $\log_{10}$.

Table 12

| Sample # | Formulation | $\log_{10}$ survivor (CFU/mL) 20/30 s | log reduction @ 20 s | log reduction @ 30 s |
|---|---|---|---|---|
| 118 | **pH 7 carbonate buffer control** | 7.2 | ---- | ---- |
| 119 | 10% SLES + 0.1 ppm $Ag^+$ | 3.3 | 3.9 | 3.9 |
| 120 | SLES/0.1 ppm $Ag^+$ + 1 % PAA | 6.5/6.2 | 0.7 | 1.0 |
| 121 | SLES/$Ag^+$/PAA + 0.5 M $NaNO_3$ | 5.5/5.3 | 1.7 | 1.9 |
| 122 | | | | |
| 123 | SLES/0.2 ppm $Ag^+$ + 1% PAA | 5.8/5.2 | 1.4 | 2.0 |
| 124 | SLES/$Ag^+$/PAA + 0.5 M $NaNO_3$ | 5.2/4.6 | 2 | 2.6 |
| 125 | | | | |
| 126 | SLES/1 ppm $Ag^+$ + 1% PAA | 4.3/3.9 | 2.9 | 3.3 |
| 127 | SLES/$Ag^+$/PAA + 0.5 M $NaNO_3$ | 3.3/3.3 | 3.9 | 3.9 |

The biocidal efficacy of 10% SLES at 0.1-1 ppm $Ag^+$ was reduced by 3 to 1 $\log_{10}$ units with addition of 1% polycarboxylic acid PAA. However, the inclusion of $NaNO_3$ partially restored this biocidal efficacy. Without being bound by theory, it is likely that nitrate ions successfully competed with the polycarboxylic acid for silver ions. Evidence for this hypothesis is the fact that the synergy improves (larger log reduction) as the total level of silver is increased at fixed polycarboxylate level.

**Example 9. Breadth of the Nitrate Benefit in pH Space**

Table 13 provides additional examples of the benefit of nitrate salts on maintaining the enhanced biocidal action of surfactant plus silver ion.

Table 13 shows all data for formulations at 25 °C. All results were the $\log_{10}$ of surviving *S. aureus* after 20, 30 seconds exposure and were the average of 3 trials with standard deviation of 0.5 $\log_{10}$ units. The detection limit in all cases was 3 $\log_{10}$.

Table 13

| Sample # | Formulation | $\log_{10}$ survivor (CFU/mL) 20/30 s | log survivor @ 20 s | log survivor @ 30 s |
|---|---|---|---|---|
| 128 | **pH 10 carbonate buffer** | 7.5/7.5 | ---- | ---- |
| | | | | |
| 129 | 10% SLES-1EO | 7.2/7.1 | 0.3 | 0.4 |
| 130 | 5% SLES-1EO/5% Na $C_{10}$ soap | 7.3/7.3 | 0.2 | 0.2 |
| 131 | 5% 1EO/5% $C_{10}$ + 0.3 ppm $Ag^+$ | 3.3/3.3 | 4.2 | 4.2 |
| 132 | 5% 1EO/5% $C_{10}$/$Ag^+$ + 0.1 M NaCl | 5.2/4.6 | 2.3 | 2.9 |
| 133 | 1EO/$C_{10}$/$Ag^+$/NaCl + 0.5 M $NaNO_3$ | 4.7/4.0 | 2.8 | 3.5 |
| | | | | |
| 134 | 1% SLES-3EO | 7.5/7.5 | 0.0 | 0.0 |
| 135 | 0.5% SLES-3EO/0.5% Na $C_{10}$ soap | 7.5/7.5 | 0.0 | 0.0 |
| 136 | 0.5% 3EO/0.5% $C_{10}$ + 0.3 ppm $Ag^+$ | 3.713.3 | 3.8 | 4.2 |

(continued)

| Sample # | Formulation | $\log_{10}$ survivor (CFU/ mL) 20/30 s | log survivor @ 20 s | log survivor @ 30 s |
|---|---|---|---|---|
| 137 | 0.5%3EO/0.5%Cw/Ag$^+$+0.1 M NaCl | 7.1/6.2 | 0.4 | 1.3 |
| 138 | 3EO/C$_{10}$/Ag$^+$/NaCl + 0.5 M NaNO$_3$ | 6.6/5.7 | 0.9 | 1.8 |
| | | | | |
| 139 | 10% Na Decanoate | 7.4/7.4 | 0.1 | 0.1 |
| 140 | 10% Na Decanoate + 1 ppm Ag$^+$ | 3.3/3.3 | 4.2 | 4.2 |
| 141 | 10% NaC$_{10}$/Ag$^+$ + 0.03 M NaCl | 5.2/4.3 | 2.3 | 3.2 |
| 142 | 10% NaC$_{10}$/Ag$^+$/NaCl+0.5 M NaNO$_3$ | 4.6/3.7 | 2.9 | 3.8 |
| | | | | |
| 143 | **pH 7 carbonate buffer** | 7.5/7.5 | ---- | ---- |
| 144 | 10% SLES-3EO/0.4 ppm Ag/0.1 M NaCl | 6.5/6.1 | 1.0 | 1.4 |
| 145 | 10%SLES-3EO/.4Ag/.1NaCl/0.5 M NaNO$_3$ | 5.3/4.8 | 2.2 | 2.7 |
| | | | | |
| 146 | 1% SDS at pH 7 | 5.2/5.0 | 2.3 | 2.5 |
| 147 | 1% SDS + 0.3 ppm Ag+ | 3.3/3.3 | 4.2 | 4.2 |
| 148 | 1% SDS/Ag$^+$ + 0.1 M NaCl | 3.9/3.5 | 3.6 | 4.0 |
| 149 | 1% SDS/Ag/NaCl + 0.5 M NaNO$_3$ | 3.3/3.3 | 4.2 | 4.2 |
| | | | | |
| 150 | **pH 5 acetate buffer** | 7.4/7.4 | - | - |
| 151 | 1% SLES-3EO at pH 5 | 5.6/4.7 | 1.8 | 2.7 |
| 152 | 1% SLES + 0.3 ppm Ag$^+$ | 4.4/3.4 | 3.0 | 4.0 |
| 153 | 1% SLES/Ag$^+$ + 0.1 M NaCl | 5.2/4.3 | 2.2 | 3.1 |
| 154 | 1% SLES/Ag$^+$/NaCl + 0.5 M NaNO$_3$ | 4.3/3.7 | 3.1 | 3.7 |

**Claims**

1. An antimicrobial cleansing composition, comprising:

   5 to 25% by weight of a sulfate surfactant;
   0 to 5% by weight of a fatty acid soap;
   0.03 to 3 parts per million of a silver compound;
   0.05 to 1% by weight of a thickening agent; and
   1 to 10% by weight of a nitrate;
   wherein a pH of the cleansing composition is 5 to 10.

2. An antimicrobial cleansing composition, comprising:

   5 to 25% by weight of a sulfate surfactant;
   0.03 to 3 parts per million of a silver compound;
   0.05 to 1% by weight of a thickening agent;
   1 to 10% by weight of a nitrate; and
   0.05 to 1% by weight of a preservative;

wherein a pH of the cleansing composition is 4 to 5.5.

3. The cleansing composition of Claim 1 or Claim 2, wherein the sulfate surfactant is an anionic surfactant.

4. The cleansing composition of Claim 3, wherein the surfactant is selected from alkyl sulfate, alkyl ether sulfate, or a combination thereof, preferably, wherein the surfactant is sodium lauryl ether sulfate, more preferably, wherein the surfactant is sodium lauryl ether sulfate with 0, 1, 2, or 3 ethoxy groups.

5. The cleansing composition of any of the preceding claims, wherein the silver compound is a silver ion, preferably wherein the silver ion is selected from silver nitrate, silver acetate, silver oxide, silver sulfate, or a combination thereof, more preferably, wherein the silver compound is silver nitrate.

6. The cleansing composition of any of the preceding claims, wherein the thickening agent is an anionic polymer-based thickener, preferably, wherein the thickening agent is a polyacrylate based polymer or copolymer, or a combination thereof.

7. The cleansing composition of any of the preceding claims, wherein the nitrate comprises sodium nitrate, potassium nitrate, or a combination thereof, preferably, wherein the nitrate comprises sodium nitrate.

8. The cleansing composition of any of the preceding claims, wherein a viscosity of the cleansing composition at a shear rate of about 1 s$^{-1}$ is 1 Pascal second at a temperature of 25°C.

9. The cleansing composition of any of Claims 1 and 3 to 8, wherein the fatty acid soap is a sodium soap, preferably, wherein the fatty acid soap is a sodium soap derived from sodium dodecanoate, sodium laurate, sodium oleate, or a combination thereof.

10. The cleansing composition of any of Claims 2 to 8, wherein the preservative comprises an organic-acid based preservative, preferably wherein the organic-acid based preservative is selected from benzoic acid, salicylic acid, levulinic acid, anisic acid, or a combination thereof.

11. The cleansing composition of any of Claims 1 to 10, wherein the composition is a body cleansing or hair cleansing composition.

**Patentansprüche**

1. Antimikrobielle Reinigungszusammensetzung, umfassend:

   5 bis 25 Gewichts-% eines Sulfattensids;
   0 bis 5 Gewichts-% einer Fettsäureseife;
   0,03 bis 3 Teile pro Million einer Silberverbindung;
   0,05 bis 1 Gewichts-% eines Verdickungsmittels; und
   1 bis 10 Gewichts-% eines Nitrats;
   wobei der pH-Wert der Reinigungszusammensetzung 5 bis 10 beträgt.

2. Antimikrobielle Reinigungszusammensetzung, umfassend:

   5 bis 25 Gewichts-% eines Sulfattensids;
   0,03 bis 3 Teile pro Million einer Silberverbindung;
   0,05 bis 1 Gewichts-% eines Verdickungsmittels; und
   1 bis 10 Gewichts-% eines Nitrats; und
   0,05 bis 1 Gewichts-% eines Konservierungsmittels;
   wobei der pH-Wert der Reinigungszusammensetzung 4 bis 5,5 beträgt.

3. Reinigungszusammensetzung nach Anspruch 1 oder Anspruch 2, wobei das Sulfattensid ein anionisches Tensid ist.

4. Reinigungszusammensetzung nach Anspruch 3, wobei das Tensid unter Alkylsulfat, Alkylethersulfat oder einer Kombination davon ausgewählt ist, wobei das Tensid vorzugsweise Natriumlaurylethersulfat ist, wobei das Tensid besonders bevorzugt Natriumlaurylethersulfat mit 0, 1, 2 oder 3 Ethoxygruppen ist.

5. Reinigungszusammensetzung nach einem der vorhergehenden Ansprüche, wobei die Silberverbindung ein Silberion ist, wobei das Silberion vorzugsweise aus Silbernitrat, Silberacetat, Silberoxid, Silbersulfat oder einer Kombination davon ausgewählt ist, wobei die Silberverbindung besonders bevorzugt Silbernitrat ist.

6. Reinigungszusammensetzung nach einem der vorhergehenden Ansprüche, wobei das Verdickungsmittel ein anionisches Verdickungsmittel auf Polymerbasis ist, wobei das Verdickungsmittel vorzugsweise ein Polymer oder Copolymer auf Basis von Polyacrylat oder eine Kombination davon ist.

7. Reinigungszusammensetzung nach einem der vorhergehenden Ansprüche, wobei das Nitrat Natriumnitrat, Kaliumnitrat oder eine Kombination davon umfasst, wobei das Nitrat vorzugsweise Natriumnitrat umfasst.

8. Reinigungszusammensetzung nach einem der vorhergehenden Ansprüche, wobei eine Viskosität der Reinigungszusammensetzung bei einer Scherrate von 1 s$^{-1}$ 1 Pascal-Sekunde bei einer Temperatur von 25°C beträgt.

9. Reinigungszusammensetzung nach einem der Ansprüche 1 und 3 bis 8, wobei die Fettsäureseife eine Natriumseife ist, wobei die Fettsäureseife vorzugsweise eine Natriumseife ist, die von Natriumdodecanoat, Natriumlaurat, Natriumoleat oder einer Kombination davon abgeleitet ist.

10. Reinigungszusammensetzung nach einem der Ansprüche 2 bis 8, wobei das Konservierungsmittel ein Konservierungsmittel auf Basis einer organischen Säure ist, wobei das Konservierungsmittel auf Basis einer organischen Säure vorzugsweise unter Benzoesäure, Salicylsäure, Levulinsäure, Anisinsäure oder einer Kombination davon ausgewählt ist.

11. Reinigungszusammensetzung nach einem der nach Ansprüche 1 bis 10, wobei die Zusammensetzung eine Körperreinigungs- oder Haarreinigungszusammensetzung ist.


**Revendications**

1. Composition de nettoyage antimicrobienne, comprenant :

   5 à 25 % en masse d'un tensioactif de sulfate ;
   0 à 5 % en masse d'un savon d'acide gras ;
   0,03 à 3 parties par million d'un composé d'argent ;
   0,05 à 1 % en masse d'un agent épaississant ; et
   1 à 10 % en masse d'un nitrate ;
   dans laquelle un pH de la composition de nettoyage est de 5 à 10.

2. Composition de nettoyage antimicrobienne, comprenant :

   5 à 25 % en masse d'un tensioactif de sulfate ;
   0,03 à 3 parties par million d'un composé d'argent ;
   0,05 à 1 % en masse d'un agent épaississant ;
   1 à 10 % en masse d'un nitrate ; et
   0,05 à 1 % en masse d'un conservateur ;
   dans laquelle un pH de la composition de nettoyage est de 4 à 5,5.

3. Composition de nettoyage selon la revendication 1 ou revendication 2, dans laquelle le tensioactif de sulfate est un tensioactif anionique.

4. Composition de nettoyage selon la revendication 3, dans laquelle le tensioactif est choisi parmi un sulfate d'alkyle, sulfate d'alkyléther, ou une combinaison de ceux-ci, de préférence, dans laquelle le tensioactif est le lauryléther sulfate de sodium, encore mieux, dans laquelle le tensioactif est le lauryléther sulfate de sodium avec 0, 1, 2, ou 3 groupes éthoxy.

5. Composition de nettoyage selon l'une quelconque des revendications précédentes, dans laquelle le composé d'argent est un ion d'argent, de préférence dans laquelle l'ion d'argent est choisi parmi le nitrate d'argent, acétate d'argent, oxyde d'argent, sulfate d'argent, ou une combinaison de ceux-ci, encore mieux, dans laquelle le composé d'argent est le nitrate d'argent.

6. Composition de nettoyage selon l'une quelconque des revendications précédentes, dans laquelle l'agent épaississant est un épaississant à base de polymère anionique, de préférence, dans laquelle l'agent épaississant est un polymère ou copolymère à base de polyacrylate, ou une combinaison de ceux-ci.

7. Composition de nettoyage selon l'une quelconque des revendications précédentes, dans laquelle le nitrate comprend du nitrate de sodium, nitrate de potassium, ou une combinaison de ceux-ci, de préférence, dans laquelle le nitrate comprend du nitrate de sodium.

8. Composition de nettoyage selon l'une quelconque des revendications précédentes, dans laquelle une viscosité de la composition de nettoyage à une vitesse de cisaillement de 1 s$^{-1}$ est de 1 Pascal seconde à une température de 25°C.

9. Composition de nettoyage selon l'une quelconque des revendications 1 et 3 à 8, dans laquelle le savon d'acide gras est un savon de sodium, de préférence, dans laquelle le savon d'acide gras est un savon de sodium dérivé de dodécanoate de sodium, laurate de sodium, oléate de sodium, ou d'une combinaison de ceux-ci.

10. Composition de nettoyage selon l'une quelconque des revendications 2 à 8, dans laquelle le conservateur comprend un conversateur à base d'acide organique, de préférence dans laquelle le conservateur à base d'acide organique est choisi parmi l'acide benzoïque, acide salicylique, acide lévulinique, acide anisique, ou une combinaison de ceux-ci.

11. Composition de nettoyage selon l'une quelconque des revendications 1 à 10, dans laquelle la composition est une composition pour le nettoyage du corps ou le nettoyage des cheveux.

# Fig. 1

Plated *S. aureus* (ATCC 6538), pH = 7

pH buffer ——

10% SLES-1EO – –✳– –

1 ppm Ag+ – ·⊙· –

10% SLES, 1 ppm Ag+ – – –△– – –

# Fig. 2

× NaCl

☐ NaNO3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6764988 B, D.W. Koenig
- US 20160053206 A1, P. Chandar
- US 20160362646 A1, Agarkhed
- US 5393466 A, Ilardi
- US 5389279 A, Au
- US 5009814 A, Kelkenberg
- EP 556957 A

- US 6846787 B, Farrell
- US 6849585 B, Farrell
- US 7351682 B, Moaddel
- US 20110224120 A1
- US 20120034314 A1
- US 20160362646 A
- US 20120034314 A

### Non-patent literature cited in the description

- **D.G. ROMERO-URBINA et al.** Ultrastructural change in methicillin-resistant Staphylococcus aureus induced by positively charged silver nanoparticles. *Beilstein Journal of Nanotechnology,* 2015, vol. 6, 2396-2405
- **R.E. BURRELL.** A scientific perspective on the use of topical silver preparations. *Ostomy/Wound Management,* 2003, vol. 49, 19-24
- **A.B.G. LANSDOWN ; A. WILLIAMS.** How safe is silver in wound care. *J. Wound Care,* 2004, vol. 13, 131-136
- **Q.L. FENG et al.** A mechanistic study of the antibacterial effect of silver ions on Escherichia coli and Staphylococcus aureus. *J. Biomedical Materials Research,* 2000, vol. 52, 662-668
- **C.P. RANDALL et al.** The silver cation (Ag+): anti-staphylococcal activity, mode of action and resistance studies. *J. Antimicrobial Chemotherapy,* 2013, vol. 68, 131-138
- **KALIA et al.** Nanofibrillated cellulose: surface modification and potential applications. *Colloidal Polymer Science,* 2014, vol. 292, 5-31
- Preservation of cosmetics and toiletries. **G. HILL.** Handbook of Bioicides and Preservatives Use. Springer Science and Business, 1995
- **D. KOLEY ; A.J. BARD.** *PNAS,* 2010, vol. 107, 16783-16787

- **M. NAZARI et al.** Classifying surfactants with respect to their effect on lipid membrane order. *Biophysical Journal,* 2012, vol. 102, 498-506
- **M.J. FEVOLA.** Sodium Lauroamphoacetate. *Cosmetics & Toiletries,* 2011, vol. 126, 844-848
- Guidelines for drinking-water quality. Health criteria and other supporting information. World Health Organization, 1996, vol. 2
- **S. HERRWERTH et al.** Highly concentrated Cocamidopropyl betaine - The latest developments for improved sustainability and enhanced skin care. *Tenside,* 2008, vol. 45, 304-308
- **B. JONSSON et al.** Surfactants and Polymers in Aqueous Solution. John Wiley & Sons, 1998
- **R. RANGANATHAN et al.** Surfactant- and salt-induced growth of normal sodium alkyl sulfate micelles well above their critical micelle concentrations. *J. Phys. Chem,* 2000, vol. 8 (104), 2260-2264
- **V. SAMBHY ; M.M. MACBRIDE ; B.R. PETERSON ; A. SEN.** Silver bromide nanoparticle/polymer composites: Dual action tunable antimicrobial materials. *J. Am. Chem. Soc.,* 2006, vol. 128, 9798-9808
- **M. YANG ; J.-G. ZHAO ; J.-J. LI.** Synthesis of porous spherical AgBr nanoparticles in the presence of gelatin using AgCl as the precursor. *Colloids and Surfaces A,* 2007, vol. 295, 81-84